# EUROPEAN PATENT APPLICATION

(11) **EP 2 320 231 A2**
(43) Date of publication of application: **11.05.2011**
(21) Application number: 10184879.4
(22) Date of filing: 21.01.2000
(51) Int. Cl.: G01N 33/543

(54) **Assays involving sam-modified colloids and non-colloidal structures**

(30) Priority: 07.05.1999 US 133148 P; 03.05.1999 US 132289 P; 23.01.1999 US 116975 P
(62) Divisional of application: 00911609.6
(71) Applicant: Minerva Biotechnologies Corporation, Newton, MA 02458 (US)
(72) Inventor: Bambad, Cynthia, Newton, MA 02458 (US); Bambad, Shoshana, Newton, MA 02458 (US)
(74) Representative: Cowley, Catherine Mary

(57) **Abstract**

The invention provides novel techniques for derivatizing colloids with self-assembled monolayers. This provides the capability of a wide variety of assays including chemical or biochemical agent/agent interaction studies. Bio-derivatized colloids, with or without signaling entities, are used to probe interactions with species on non-colloidal structures. The invention provides techniques for immobilizing colloidal particles on a wide variety of non-colloidal structures. Included is the ability to decorate a variety of non-colloidal structures including beads, with colloids as a detectable assay. This allows, in many cases, assays detectable via the unaided human eye, as well as assays detectable via automated determination of a change of interaction of electromagnetic radiation with the colloids, e.g., absorption, light-scattering, and the like.

## Description

### Field of the Invention

This invention relates generally to chemical and biochemical detection methods, and more particularly to techniques in which colloids bind to non-colloidal structures such as electrodes, beads, or cells, and to techniques in which multiple signals indicate a single binding event. Techniques including drug screening are facilitated by the invention.

### Background of the Invention

Drug discovery is facilitated by screening large numbers of candidate compounds for interaction with target receptor or proteins under physiological conditions. Of particular importance are cell surface receptors or proteins. Many of the biomolecular interactions that promote tumorigenesis involve cell surface proteins that mediate both intra- and intercellular signaling. "Tumor markers" are molecules on the surface of a cell that are either exclusively expressed, or over-expressed, as a result of transformation to a neoplastic state. Some of these markers have been correlated with the ability of the tumor to invade tissues and display an aggressive course of growth characterized by metastases (these tumors generally are associated with a poor outcome for the patient). For example, the interaction between the cell surface receptor αVβ3 and the cell adhesion molecule vitronectin has been implicated in angiogenesis. *See* J. Varner et al., "Integrins and cancer," Curr Opin Cell Biol. 8:724 (1996); B. Vailhe et al., "In vitro angiogenesis is modulated by the mechanical properties of fibrin gels and is related to a αVβ3 integrin localization," In Vitro Cell Dev Biol Anim., 33:763 (1997); M. Horton, "The αVβ3 integrin 'vitronectin receptor'," Int J Biochem Cell Biol, 29:721 (1997). Indeed, the increased concentration of this receptor on a melanoma cell has been correlated with a poor prognosis. Another example of a cell surface receptor that promotes tumorigenesis and/or angiogenesis is the MUC-1 antigen; this antigen is overexpressed on breast, prostate, lung and ovarian cancers.

The search for drugs to bind to and block cell surface receptors implicated in tumorigenesis has been technically challenging because there are few assays that work with intact cells. The ability to detect interactions between ligands and target receptors on the surface of live, intact cells, would enable the screening of candidate compounds to disrupt these interactions. Screening compound libraries for drugs that inhibit the action of cell surface receptors depends critically on the receptors being in their native conformation and multimerization state throughout the drug screening process. According to current technologies, it is difficult or impossible to detect the interaction of cell surface receptors with their natural ligands.

Current technologies are limited in their ability to assay ligand interactions on cells. Fluorescence activated cell sorting (FACS) is one of the few techniques that enables the detection of cell surface receptors. One complication in using this technique to screen for drugs to block cell surface receptors is that: 1) the technique is sequential and cannot be readily multiplexed to facilitate massive drug screening; and 2) the technique is only feasible for detecting *antibodies* bound to the receptors; antibody antigen interactions are typically high affinity interactions that cannot be disrupted by drugs. According to current technologies, it is difficult or impossible to detect the interaction of cell surface receptors with their natural ligands. Screening compound libraries for drugs that inhibit the action of cell surface receptors depends critically on the receptors being in their native conformation and multimerization state, on the cell surface, throughout the drug screening process.

Another technique that can be used to query cell surface receptors is an ELISA assay. In this technique, cells are adhered to a 96-well plastic plate. A cognate antibody is allowed to bind to the cell surface receptor of interest and unbound antibody is washed away. The availability of the receptor is inferred by detecting the presence of the cognate antibody. The presence of bound antibody is detected by introducing a second antibody, conjugated to a detectable label, which is active against the *species* of the cognate antibody. There are several inherent problems which limit the usefulness of this technique for assessing the availability of cell surface proteins or for screening for drugs to block them: 1) the technique is plagued by false positives that result from the non-specific adsorption of antibodies to the plastic; 2) ligand-receptor interactions are often disrupted by the many washing steps; and most importantly, 3) antibodies rather than natural ligands must be used in this assay; because these are typically high affinity interactions, they cannot be readily disrupted by drugs that bind the receptor; additionally, unlike the natural ligand, the antibody may bind the target receptor at a site that does not disrupt the receptor's normal function.

What is needed is an approach for monitoring or controlling binding events between chemical or biological species that increases versatility and can increase sensitivity in a wide variety of specific interactions. For example, an approach for screening drugs against specific interactions of cell receptors and their ligands (including but not limited to the interaction of the (αVβ3 receptor) would be advantageous. In the latter example, the assay should be flexible so as to permit screening against a wide variety of tumor types, and particularly invasive tumors. Importantly, the screening approach should permit the rapid screening of large numbers of candidate drugs.

### Summary of the Invention

The present invention provides a variety of novel methods, compositions and species, and articles for monitoring (detecting) interactions between chemical or biological species including techniques useful for drug screening. Major aspects of the present invention include the following. Tools for proteomic studies including protein chips and particles for signaling interactions, and multi-particle systems such as two-particle systems. In multi-particle systems one particle can be a recruitable particle and the other particle can carry a binding partner of an agent presented by the recruitable particle and can also be a signaling entity or carry an auxiliary signaling entity. Another major area involves cell studies, especially techniques involving interactions between ligands and cell surface proteins and receptors. Discovery and therapeutics involving drugs that can effect these interactions also is described, with an emphasis on drug therapy involving angiogenesis. Specifically, cell receptor/ligand interactions that can inhibit or promote angiogenesis are described. Another area involves detecting proteins, either in solution or on the surfaces of intact cells, for diagnostic purposes.

In one aspect, the invention provides a method in which a colloid particle is given the ability to fasten to a non-colloidal structure, and then whether the colloid particle has fastened to the non-colloidal structure is determined. This aspect covers a wide range of interactions involving colloid particles or, preferably, many colloid particles with a variety of structures. Non-colloidal structures including beads, such as magnetic beads, can interact with colloid particles as an aid in determining interaction of species immobilized with respect to the bead with species immobilized with respect to the colloid particles. The colloid particles can "decorate" the beads, rendering them visibly identifiable as an indication of a particular species/species interaction. Alternatively, magnetic beads can be used to recruit colloid particles to a surface where identification of the particles can be made through electronic or electrochemical or other means, and the like. Non-colloidal structures such as electrodes can act with colloid particles or species immobilized on the colloid particles to indicate a particular assay result. Non-colloidal structures such as cells can interact with colloid structures to indicate binding of the cell to a species carried by the colloid particle. Advantages of these and other techniques involving colloid particles and a non-colloid structure will become even more apparent from the description that follows.

In another aspect the invention provides a technique for linking a chemical or biological agent to an article, such as a bead, colloid particle or the like, via a metal binding tag/metal chelate linkage. Articles linked in this way to chemical or biochemical species are included in the invention.

Another aspect of the invention involves a technique for the detection of ligand/protein interaction involving providing a ligand in fixed proximal relationship with an electroactive entity and determining interaction of the ligand with the protein by monitoring an electroactive signal given by the electroactive entity where the signal is dependent upon proximity of electroactive entity to the protein. This technique can be facilitated by an article of the invention which defines a first protein immobilized at a first surface, and a ligand suspected of interacting with the protein immobilized at a second surface, which may be a particle, along with an electroactive entity which also is immobilized at the second surface. This aspect of the invention facilitates one further feature of the invention that is a method for determining protein/ligand interaction in the absence of surface plasmon resonance without labeling either the protein or the ligand.

The invention also provides techniques and components that allow for a method of signaling a single binding of a first biological or chemical agent to a second biological or chemical agent with a plurality of signaling entities. An article provided by the invention facilitates this technique and it defines a first biological or chemical agent capable of biological or chemical binding to a second agent, linked to a plurality of signaling entities. The plurality of signaling entities can be linked to the agent by way of a polymer or dendrimer that carries a plurality of signaling entities and is adapted for linkage to an agent. The technique also can be facilitated by immobilizing the agent at a colloid particle, e.g. at the surface of a colloid particle at which a plurality of signaling entities are also immobilized. In preferred embodiments, more than three signaling entities signal a single chemical or biological binding event simultaneously. More preferably, at least 10, more preferably at least 50, and more preferably at least 1000 or 10,000 signaling entities signal a single chemical or biological binding event simultaneously according to this aspect of the invention.

Another aspect of the invention is defined by a colloid particle that has both a signaling entity and a protein immobilized thereupon. Techniques for use of this component also are included in the invention.

The invention also provides techniques for controlling the electronic permeability across a self-assembled monolayer at a surface. In the technique, a self-assembled monolayer is formed upon a surface and is defined by a mixture of at least a first molecular species and a second molecular species. The first molecular species has a structure that promotes the self-assembly at the surface in a tightly-packed manner that prevents fluid to which the surface is exposed from communication electrically with the surface. That is, the first species forms a relatively tightly-packed self-assembled monolayer (SAM) that seals the surface against fluids for the SAM is made up of only the first species. The SAM according to this aspect of the invention is made up of the first species and a second species that has a different molecular structure where the molecular structure causes disruption of the tightly-packed self-assembled structure thereby defining defects in the tightly-packed structure that allow fluid to which the surface is exposed to communicate electrically with the surface. In this context, the fluid communicates electrically with the surface by contacting the surface or coming in close enough proximity to the surface that electronic communication, via tunneling or the like, can occur. As will be seen from the description that follows, this aspect of the invention can be very useful in conjunction with other techniques of the invention.

As mentioned, one feature of the invention is that it provides a series of components and techniques for drug screening. The approach provides 1) a modular system for the attachment of natural ligands to universal signaling elements; 2) enhanced sensitivity of detection through the attachment of a plurality of signaling elements to each ligand; 3) a simpler format (without the need for washing steps, enzymatic cleavage and toxic substrates); 4) a convenient electronic output; and 5) the capability for multiplexing.

In one embodiment of the assay, the present invention contemplates immobilizing live, intact growing cells or cell-derived molecules, on conducting solid supports. Such cells or cell-derived molecules, having both surface molecules (e.g. receptors) and intracellular signaling proteins, are attached to solid supports that can either be surfaces or particle-like in nature. Ligands (or "binding partners") to these cells or cell-derived molecules, which can include both known and unknown ligands as well as putative drug candidates that are either unattached to other solid supports or attached to surfaces or particle-like structures, are allowed to interact with other cell-derived molecules in a manner such that binding between the two binding partners occurs and can be detected. One of the binding partners or its attached support can additionally be derivatized with a substance that can be recognized and quantitated by a detection apparatus. This complex (through interaction) is then brought into the presence of the detection apparatus using characteristics of the associated complex that differentiate it from the unassociated binding partners.

In one embodiment, the cells are grown on a metal support (e.g. gold) that has been treated (e.g. coated with molecules compatible with cell growth) in a manner that preserves the conducting nature of the metal support. In one embodiment, a homogeneous conducting layer of molecules is attached to a metal surface that allows the conduction of electrons at a rate that is higher than a metal uniformly coated with insulating species of molecules (such as saturated alkyl thiolates). In another embodiment, a heterogeneous layer of molecules including a mixture of first a second species that allow fluid exposed to the surface to communicate electrically with the surface as described above. In another embodiment, a heterogeneous conducting layer of molecules is attached to a metal surface; the heterogenous layer can be viewed as a lawn of low or non-conducting species (e.g. one such species or a plurality of such species) interrupted by conducting or high-conducting molecules (or "defects" in the lawn).

In a preferred embodiment of a heterogeneous conducting layer or heterogeneous layer allowing defects promoting fluid/surface electrocommunication, two types of molecules are attached to a metal surface: 1) alkyl thiol spacer molecules (terminated with either ethylene glycol units or methyl groups to inhibit non specific adsorption) are used to form the lawn and 2) a conducting species or defect-forming species. A conducting species can be readily identified by the experiments described below. In one embodiment, the conducting species contains at least one ring structure (e.g. a benzene ring, a pyrimidine ring, etc.). In another embodiment, the conducting species contains no ring structure, but contains a group selected from -O-, -OH-, -NH₂- and SH.

In one embodiment of a homogeneous conducting layer, only one type of molecule is attached to a metal surface, i. e. a conducting species. Other embodiments employing a plurality of conducting species are also contemplated. Further description of defect-forming molecules and other aspects of this arrangement are described below.

As noted above, the present invention contemplates both methods and compositions. In one embodiment, the present invention contemplates a composition, comprising a first molecule and one or more signaling elements (i. e. molecules or clusters of molecules which provide a signal that can be detected by any means, including but not limited to, detection by change in light transmission, change in solution color, change in charge, etc.) attached to a solid support, wherein said first molecule is a ligand and is capable of interacting with a protein or cell-surface receptor. In another embodiment, the present invention contemplates a composition, comprising a first molecule, a second molecule and a third molecule attached to a solid support, wherein said first molecule comprises a ligand capable of interacting with a protein or cell-surface receptor, wherein said second molecule forms a monolayer on said solid support, and wherein said third molecule is electroactive.

It is not intended that the present invention be limited by the nature of the solid support. In one embodiment, the solid support is a colloid (e.g. gold colloid). It is also not intended that the present invention be limited by the nature of attachment of the ligand to the solid support. In one embodiment, said ligand is covalently attached (directly or through another ligand or binding moiety) to the solid support. In another embodiment, the ligand is attached non-covalently or by electrostatic or ionic interaction.

The present invention contemplates a variety of signaling elements, including but not limited to fluorescent molecules and enzymes capable of acting on color-producing substrates. In a preferred embodiment, the present invention contemplates electroactive molecules that is, molecules having an oxidation/reduction potential that can be determined electronically or electrochemically proximate a working electrode of an appropriate, conventional electrical arrangement, as signaling elements. A preferred electroactive molecule is a metallocene. Metallocenes that can operate as electroactive signaling elements in accordance with the invention are known. One example of a particularly preferred electroactive molecule is one containing a ferrocene or a ferrocene derivative group or derivative, such as ferrocenyl thiol (C₃₅H₂₄FeS); however, other organic complexes of transitions metals are also contemplated as signaling elements.

It is not intended that the present invention be limited by the nature of the chemical or biochemical agent. A wide variety of agents and binding partners of those agents such as protein/protein, protein/peptide, antibody/antigen, antibody/hapten, enzyme/substrate, enzyme/inhibitor, enzyme/cofactor, binding protein/substrate, carrier protein/substrate, lectin/carbohydrate, receptor/hormone, receptor/effector, complementary strands of nucleic acid, protein/nucleic acid repressor/inducer, ligand/cell surface receptor, virus/ligand, etc., can be used for binding interactions of the inventions. In one embodiment, the agent is a ligand, specifically a peptide. In a preferred embodiment, the peptide is derivatized with a moiety (such as a histidine tag) that can bind to a metal chelate. In this embodiment, it is convenient that the solid support comprise a metal chelate and said peptide is attached to said solid support via binding of said moiety to said metal chelate.

In some embodiments, cell-derived molecules, including both cell-surface receptors and intracellular signaling proteins, exist on or are attached to solid supports that can either be surfaces or particle-like in nature. Binding partners of these cell-derived proteins, which can include both known and unknown ligands as well as putative drug candidates, are attached to surfaces and/or particle-like structures, and are allowed to interact with the cell-derived proteins in a manner such that binding between the two binding partners occurs. One of the binding partners or its attached support can additionally be derivatized with a detectable substance. Interacting complexes are identified using characteristics of the associated complex that differentiate it from the unassociated binding partners. The presence of, or a change in, a detectable moiety, that is either co-immobilized with one of the binding partners on a common solid support or directly attached to one of the binding partners, is detected. Molecules that disrupt a relevant interaction can be identified by detecting a loss of this signal. Interacting partners are brought to a sensing apparatus by confining one of the binding partners to the sensing area and allowing it to recruit the other binding partner, or by manipulating characteristics of the associated complex that differentiate it from the unassociated binding partners, or by attaching a recruitable element to one of the binding partners or its associated solid support.

For example, a chemical or biological binding partner can be attached to the surface of a solid support, which can be an electrode, a substantially planar article such as a common biotechnology chip, or the like. A binding partner of the chemical or biological agent can be attached to a colloid, and exposure of the colloid to the surface of the article results in binding of the binding partners thus immobilization of a colloid at the surface. This can be determined by determination of the colloid at the surface, specifically at a particular location of a surface. The colloid particle alternatively can include an auxiliary signaling entity that facilitates this determination (such as an electroactive signaling entity or a visual signaling entity such as a fluorescent tag).

Specifically, cell-derived molecules, such as proteins are attached to surfaces and ligands, as well as putative drug candidates are attached to particles, and are allowed to interact with the cell-derived proteins in a manner such that binding between the two binding partners occurs. One of the binding partners or its attached support can be derivatized with a detectable substance. Interacting partners are brought to a sensing apparatus by confining one of the binding partners to a sensing area and allowing it to recruit the other binding partner. Alternatively, one of the binding partners can be attached to a recruitable particle that may or may not display signaling entities.

One aspect of the invention involves recruiting an electronic signaling entity to an electrode using a magnetic material. This aspect can find use in many assays and other techniques of the invention. In the method, typically, a signaling entity is provided with the ability to become immobilized relative to the magnetic material (which can be a magnetic bead). The magnetic material and signaling entity can become immobilized relative to each other via a variety of chemical and/or physical linkages described herein. For example a first species may be immobilized relative to or fastened to a magnetic material and a second species may be immobilized relative to or fastened to the signaling entity or the first and second species can bind to each other. The first and second species can be essentially any species described herein or known in the art for binding, and in one embodiment are proteins. In a preferred embodiment, the proteins are not antibodies but are, for example, a ligand and a cognate receptor, etc. The signaling entity can be fastened to an intermediate entity, such as a colloid particle, to which one of the proteins that acts as a binding partner also is fastened. A signaling entity also can be recruited to an electrode without use of a magnetic material. In this arrangement the signaling entity can be immobilized with respect to a binding partner of a species with respect to the electrode, and the binding partners can be allowed to bind to each other. Essentially any binding partner interaction as described herein or known in the art can facilitate this technique.

Other advantages, novel features, and objects of the invention will become apparent from the following detailed description of the invention when considered in conjunction with the accompanying drawings, which are schematic and which are not intended to be drawn to scale. In the figures, each identical or nearly identical component that is illustrated in various figures is represented by a single numeral. For purposes of clarity, not every component is labeled in every figure, nor is every component of each embodiment of the invention shown where illustration is not necessary to allow those of ordinary skill in the art to understand the invention.

### Brief Description of the Drawings

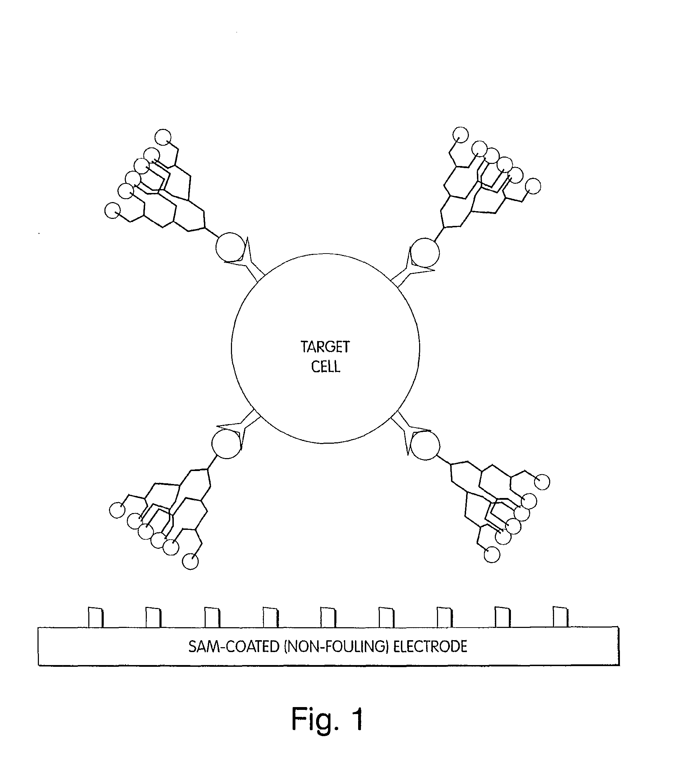

Figure 1 depicts ligands attached to a metal-containing compound, such as ferrocene, via dendrimers or polymers interacting with their cognate cell-surface receptors on intact cells.

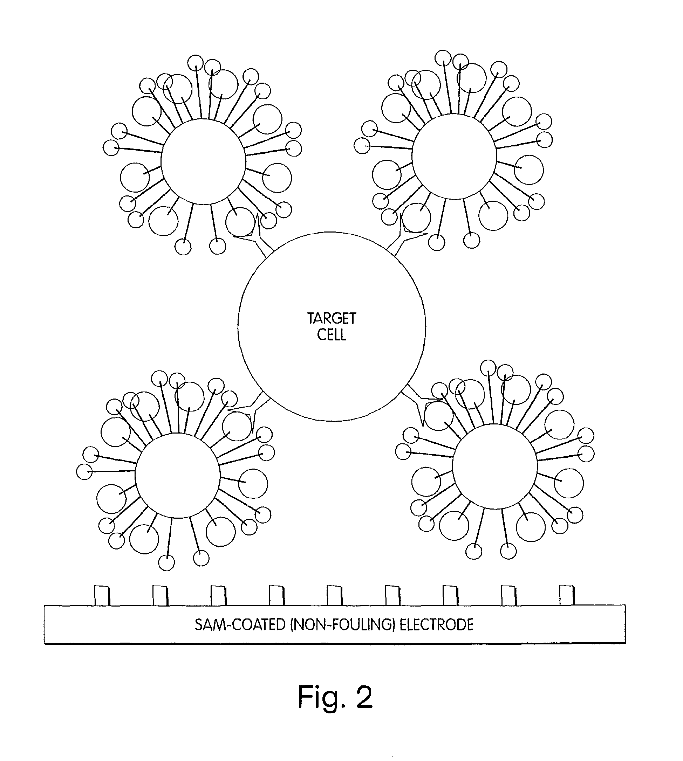

Figure 2 depicts ligands and metal-containing compounds such as ferrocene attached to particles interacting with their cognate cell surface receptors on intact cells.

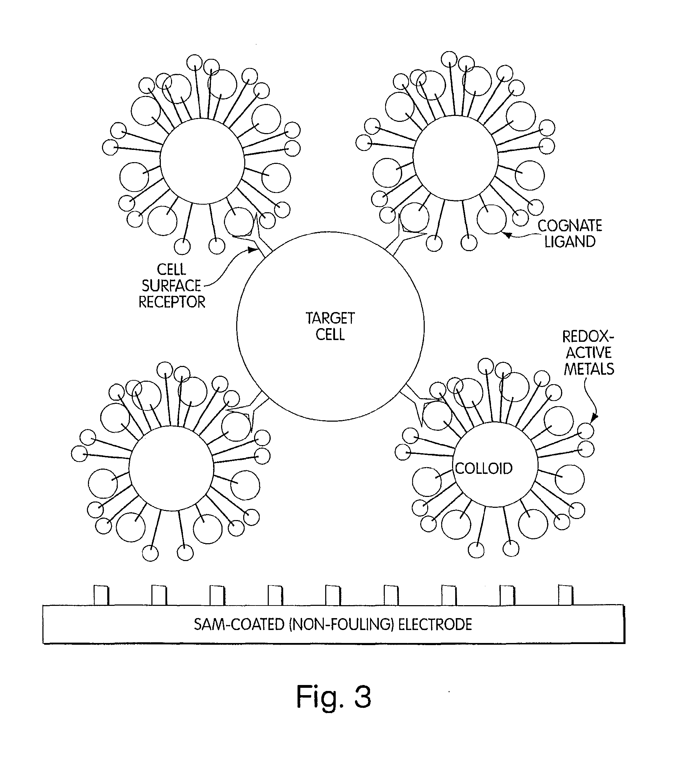

Figure 3 shows proteins attached to self-assembled monolayers on colloids that also present redox-active metals for electronic or electrochemical signaling. Cells bearing target receptors are incubated with colloids bearing a cognate ligand for the receptor of interest then electrophoretically or magnetically attracted to the sensing electrode. Note that the figure is not drawn to scale and the colloids can be made in a variety of sizes, ranging from 20 gold atoms to hundreds of nanometers in diameter.

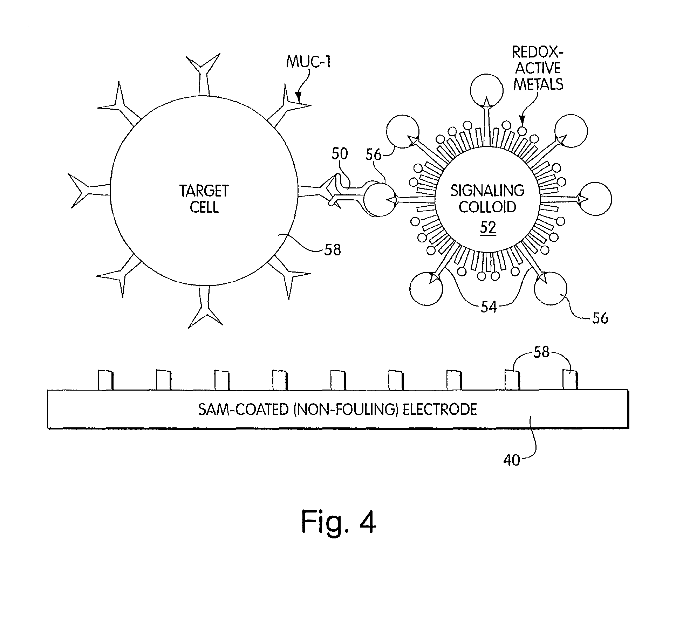

Figure 4 shows cells bearing a tumor marker MUC-1 that are incubated with electronic signaling colloids that present the MUC-1 specific antibody, DF-3. The antibody is attached to the colloid via a His-tagged protein G that is bound to NTA/Ni(II) groups that were incorporated into a self-assembled monolayer on the colloid.

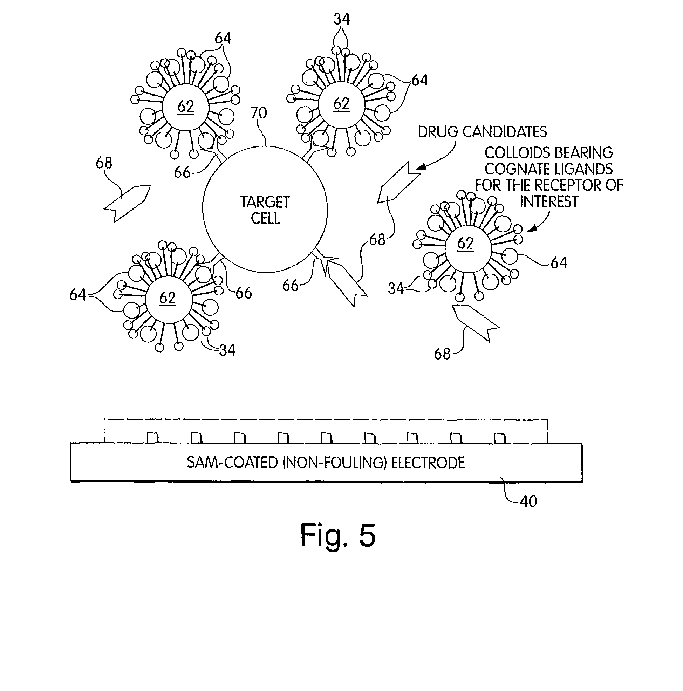

Figure 5 shows one embodiment of an assay that can be used to screen drug libraries for the ability of their individual members to disrupt interactions with cell-surface proteins.

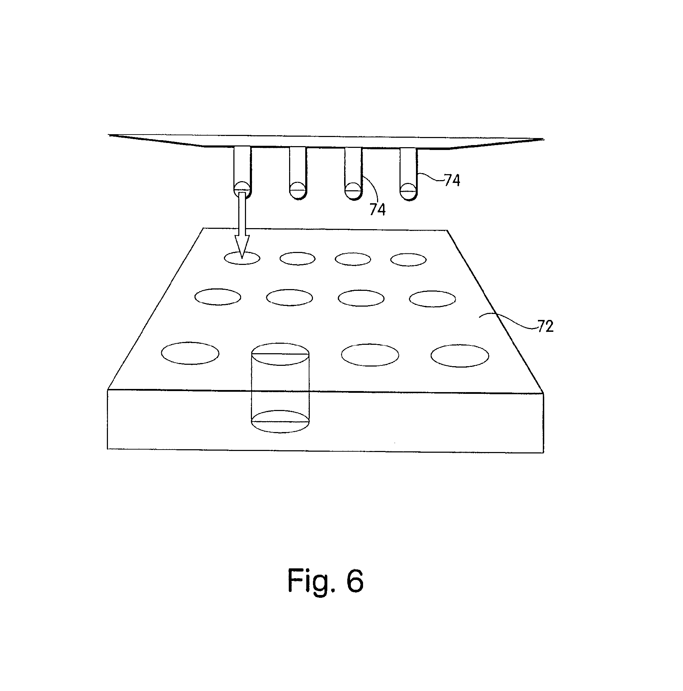

Figure 6 shows that a drug screening assay can be readily multiplexed by interfacing microwells (can be disposable) with a microelectrode array and robotics. Analysis of arrays of electrodes can be performed using a modified electrochemical analyzer capable of concurrent analysis of a plurality of electrodes. The electrodes can be configured so as to be inserted (see arrow) into the microwells.

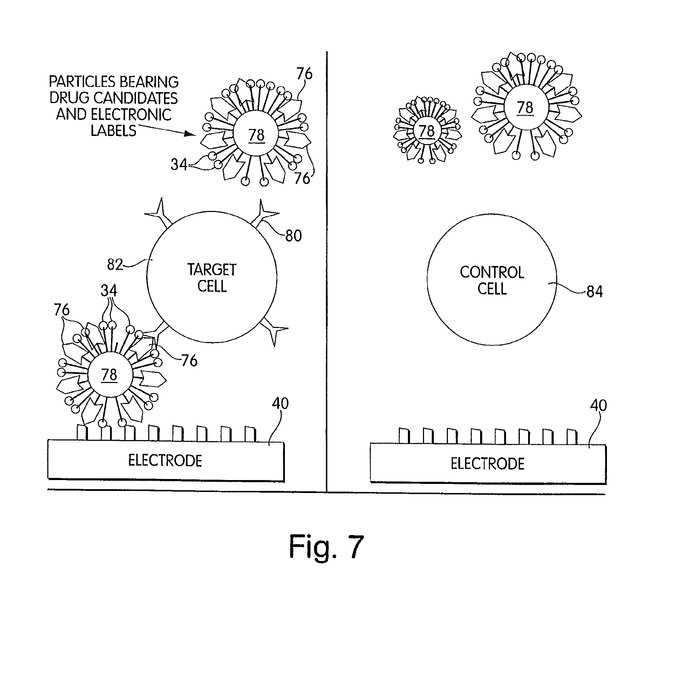

Figure 7 shows that drug candidates can be synthesized on, or covalently attached to, particles that also bear electro-active signaling moieties. Drug candidates attached to signaling particles can be incubated with cells presenting the receptor of interest and control cells that did not express the receptor of interest. A drug-target interaction in this assay will result in a gain of signal.

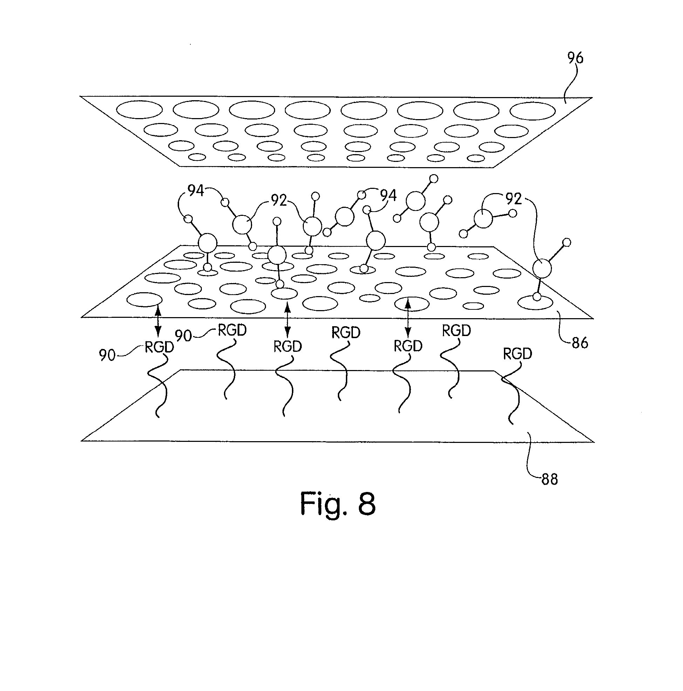

Figure 8 shows a tissue specimen that is attached to a flexible, semi-permeable support via interaction with peptides containing RGD motifs. The specimen is then incubated with electro-active signaling colloids that bear a ligand for a cell-surface receptor of interest. The specimen is rinsed, then interfaced with a microelectrode array. The electrode array can be made such that the electrode dimensions are comparable to cell dimensions. The specimen is then characterized by ACV, which can then be correlated to histopathology.

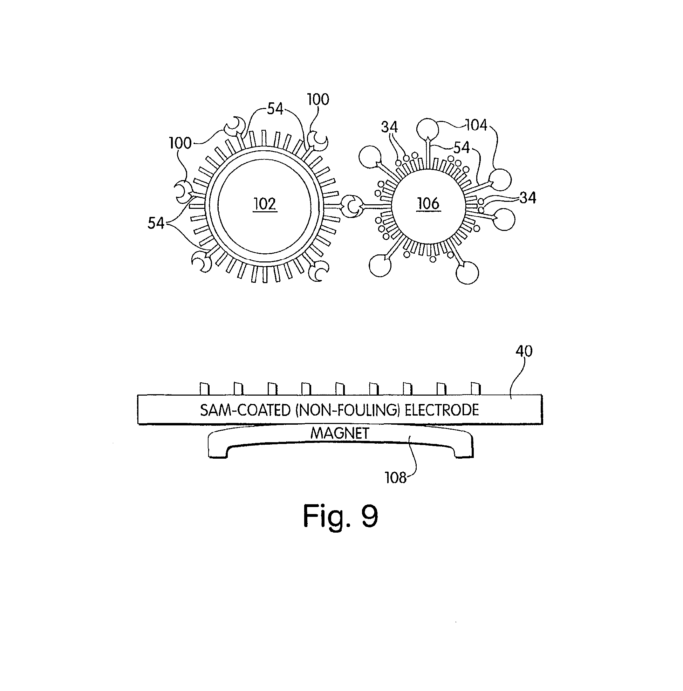

Figure 9 shows a protein-protein interaction assay that is based on universal recruitment and signaling particles that can be easily multiplexed. Self-assembled monolayers bearing NTA/Ni(II) ligands that selectively capture His-tagged proteins are formed on signaling colloids and gold-coated magnetic beads. Each particle type is then briefly incubated with a different His-tagged protein. A magnetic field then attracts the magnetic bead to the sensing electrode. A signal is only transduced when the electro-active labels (which can be redox-active moieties) on the colloid are brought to the electrode through an interaction between a first protein and a second protein. Note that non-His-tagged proteins can also be attached to universal particles that bear self-assembled monolayers presenting carboxy-terminated thiols.

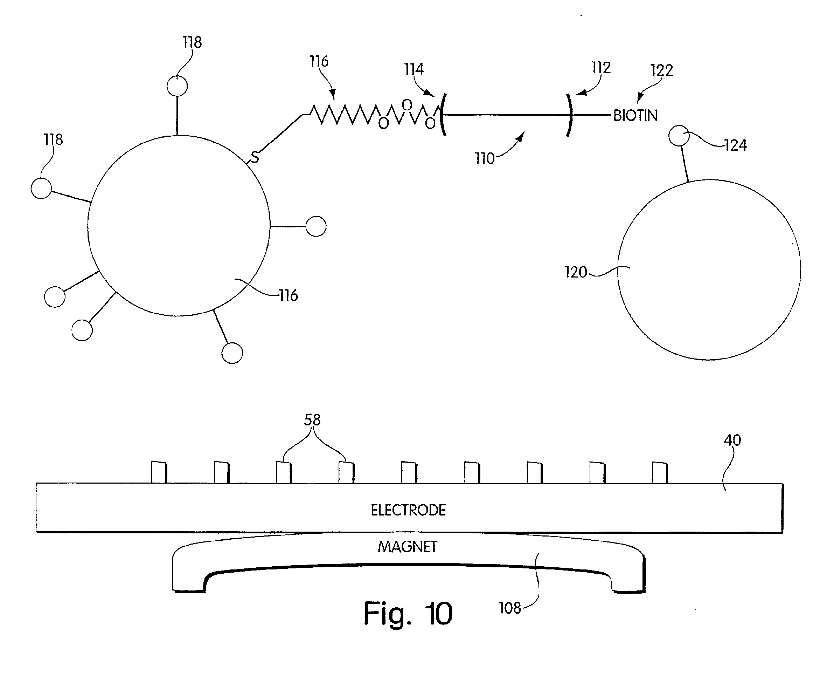

Figure 10 shows electro-active signaling colloids derivatized with thiols bearing enzyme cleavage sites (ECS) and terminated with biotin. Magnetic beads are derivatized with streptavidin, then the magnetic beads and signaling colloids are incubated with the enzyme of interest and drug candidates. If a drug candidate inhibited the action of the enzyme of interest then the site will not be cleaved and the signaling colloid will remain attached to the magnetic bead via the biotinstreptavidin interaction. When the resulting complexes are magnetically attracted to a sensing electrode, a current peak will result, making the assay a gain of signal detection assay.

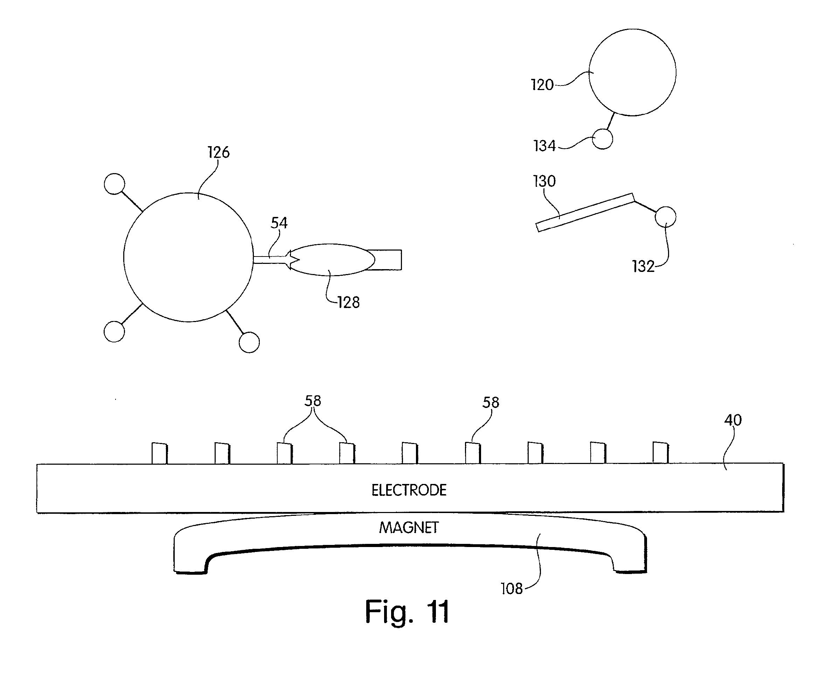

Figure 11 shows a strategy for screening for drugs that modulate enzyme activity.
   In this scheme, a protein that is a target of enzyme modification is attached to a colloid that also bears an electro-active moiety. The chemical group that the enzyme adds to the target protein has been labeled with biotin. When the enzyme functions properly, the biotin-labeled compound is added to the protein on the signaling colloid. This resulting complex is then connected to a streptavidin-coated magnetic bead. The complex is then electromagnetically attracted to a sensing electrode. Drug candidates can be added to the assay. A loss of signal compared to controls would indicate that the drug candidate inhibited the enzyme's activity while a gain of signal would indicate that a drug enhanced the enzyme's activity.

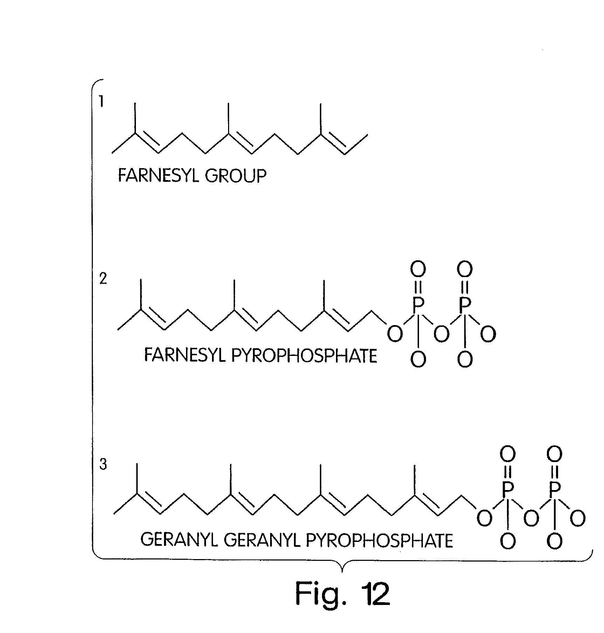

Figure 12 shows the chemical structures of the farnesyl group, farnesyl pyrophosphate, and geranyl-geranyl pyrophosphate.
Figure 13 shows the modification of farnesyl- or geranyl-like moieties with a recognition group such as biotin.
Figure 14 shows farnesyl pyrophosphate derivatives bearing a biotin moiety. The farnesyl protein transferase (FPT) enzyme catalyzes the addition of the farnesyl derivative to RAS protein variants. The activity of the enzyme, or the ability of the drug candidate to modify enzyme activity can be quantitated by measuring current output when magnetic beads with streptavidin are used to recruit the complexes to the sensing electrode.
Figure 15 shows that drug candidates can be assessed for their ability to modulate enzyme activity. Each spatially addressable microwell contains a RAS protein (wild type or variant), the FPT enzyme, the biotin-modified addition group (farnesyl derivatives), magnetic beads bearing streptavidin, and plus or minus drug candidates. Electrodes can then be analyzed by a variety of electronic and electrochemical techniques, including alternating current voltammetry (ACV).
Figure 16 shows a plot of an ACV analysis of the interaction of an RGD-containing ligand with cells grown on collagen-coated electrodes.
Figure 17 shows a plot of an ACV analysis of the interaction of an (negative) control ligand with cells grown on collagen-coated electrodes.
Figure 18 shows a plot of an ACV analysis of cells grown on a metal support comprising only a single type of conductive molecule.
Figure 19 shows a plot of an ACV analysis of the interaction of an RGD-containing ligand with cells grown on a metal support comprising a heterogeneous monolayer of insulating and conducting molecules.
Figure 20 is a photocopy of a photograph of colloid-decorated beads, specifically, colloids linked to beads via protein/protein interaction.
Figure 21 is a photocopy of a photograph of the negative control of the experiment shown in Figure 20.
Figure 22 shows a plot demonstrating the increase in conductivity of the monolayer as the percent of conducting molecules (in a lawn of insulating species) is increased.
Figure 23 shows a plot of an ACV analysis of the conductivity of a monolayer of conductive self-assembled monolayer containing a two-unit molecular wire poly (ethynylphenyl) thiol, MF1 shown against a negative control of a non-conductive self-assembled monolayer, as measured with a solution of ferrocene.
Figure 24 shows a plot of an ACV analysis of the conductivity of a monolayer of the two self-assembled monolayers of Figure 23, as measured against more highly-conductive monolayers, as measured with a solution of ferrocene.
Figure 25 shows ACV analysis of protein/protein interaction as measured by binding of a colloid to a magnetic bead.
Figure 26 shows ACV demonstration of enhanced electronic communication across a self-assembled monolayer, and redox signaling of protein immobilization to a cell surface, against a control.
Figure 27 is a schematic illustration of the assay, the plot of which is shown in Figure 25, of protein/protein interaction as measured by linkage of colloid to magnetic bead.
Figure 28 shows a plot of an ACV analysis an electrode containing surface-bound proteins and their affect on ferrocene oxidation potential (solid line), and negative control (dotted line).
Figures 29A and 29B are photocopies of photomicrographs (40X magnification) of cells decorated by colloids selectively at locations where receptor was expressed (29B) and control (29A).
Figures 30A and 30B are photocopies of computer-digitized microscope images of beads carrying non-specifically attached protein decorated by colloid particles carrying, on a self-assembled monolayer, the protein's binding partner (30A) and control (30B).
Figure 31 is a bar graph showing six order of magnitude improved signaling resulting from signaling a single binding event with a plurality of signaling entities.
Figures 32A and 32B show ACV plots of an experiment demonstrating the use of gravity to recruit a signaling entity indicating a binding interaction to a surface, and a control.

### Detailed Description of the Invention

International patent application serial number PCT/US00/01997, filed 01/25/00 by Bamdad et al., entitled "Rapid and Sensitive Detection of Aberrant Protein Aggregation in Neurodegenerative Diseases", International patent application serial number PCT/US00/01504, filed 01/21/00 by Bamdad, et al, entitled "Interaction of Colloid-Immobilized Species with Species on Non-Colloidal Structures", and commonly-owned, copending U.S. patent application filed on even date herewith by Bamdad et al., entitled "Rapid and Sensitive Detection of Protein Aggregation" all are incorporated herein by reference.

### Definitions:

"Small molecule", as used herein, means a molecule less than 5 kiloDalton, more typically less than 1 kiloDalton. As used herein, "small molecule" excludes proteins.

The term "candidate drug" as used herein, refers to any medicinal substance used in humans, animals, or plants. Encompassed within this definition are compound analogs, naturally occurring, synthetic and recombinant pharmaceuticals, hormones, antimicrobials, neurotransmitters, etc. This includes any substance or precursor (whether naturally occurring, synthetic or recombinant) which is to be evaluated for use as a drug for treatment of neurodegenerative disease, or other disease characterized by aberrant aggregation, or prevention thereof. Evaluation typically takes place through activity in an assay, such as the screening assays of the present invention.

A variety of types of particles can be used in the invention. For example, "fluid suspendable particle" means a particle that can be made to stay in suspension in a fluid in which it is used for purposes of the invention (typically an aqueous solution) by itself, or can be maintained in solution by application of a magnetic field, an electromagnetic field, agitation such as stirring, shaking, vibrating, sonicating, centrifuging, vortexing, or the like. A "magnetically suspendable" particle is one that can be maintained in suspension in a fluid via application of a magnetic field. An electromagnetically-suspendable particle is one that can be maintained in suspension in a fluid by application of an electromagnetic field (e.g., a particle carrying a charge, or a particle modified to carry a charge). A "self-suspendable particle" is a particle that is of low enough size and/or mass that it will remain in suspension in a fluid in which it is used (typically an aqueous solution), without assistance of for example a magnetic field, for at least 1 hour. Other self-suspendable particles will remain in suspension, without assistance, for 5 hours, 1 day, 1 week, or even 1 month, in accordance with the invention.

"Proteins" and "peptides" are well-known terms in the art, and are not precisely defined in the art in terms of the number of amino acids that each includes. As used herein, these terms are given their ordinary meaning in the art. Generally, peptides are amino acid sequences of less than about 100 amino acids in length, but can include sequences of up to 300 amino acids. Proteins generally are considered to be molecules of at least 100 amino acids.

As used herein, a "metal binding tag" refers to a group of molecules that can become fastened to a metal that is coordinated by a chelate. Suitable groups of such molecules include amino acid sequences including, but not limited to, histidines and cysteines ("polyamino acid tags"). Metal binding tags include histidine tags, defined below.

As used herein, "chelate coordinating a metal" or metal coordinated by a chelate, refers to a metal coordinated by a chelating agent that does not fill all available coordination sites on the metal, leaving some coordination sites available for binding via a metal binding tag.

As used herein, "metal binding tag/metal/chelate linkage" defines a linkage between first and second species in which a first species is immobilized relative to a metal binding tag and a second species is immobilized relative to a chelate, where the chelate coordinates a metal to which the metal binding tag is also coordinated. U.S. Patent No. 5,620,850 of Bamdad, et al., incorporated herein by reference, describes exemplary linkages.

"Signaling entity" means an entity that is capable of indicating its existence in a particular sample or at a particular location. Signaling entities of the invention can be those that are identifiable by the unaided human eye, those that may be invisible in isolation but may be detectable by the unaided human eye if in sufficient quantity (e.g., colloid particles), entities that absorb or emit electromagnetic radiation at a level or within a wavelength range such that they can be readily detected visibly (unaided or with a microscope including an electron microscope or the like), or spectroscopically, entities that can be detected electronically or electrochemically, such as redox-active molecules exhibiting a characteristic oxidation/reduction pattern upon exposure to appropriate activation energy ("electronic signaling entities"), or the like. Examples include dyes, pigments, electroactive molecules such as redox-active molecules, fluorescent moieties (including, by definition, phosphorescent moieties), up-regulating phosphors, chemiluminescent entities, electrochemiluminescent entities, or enzyme-linked signaling moieties including horse radish peroxidase and alkaline phosphatase. "Precursors of signaling entities" are entities that by themselves may not have signaling capability but, upon chemical, electrochemical, electrical, magnetic, or physical interaction with another species, become signaling entities. An example includes a chromophore having the ability to emit radiation within a particular, detectable wavelength only upon chemical interaction with another molecule. Precursors of signaling entities are distinguishable from, but are included within the definition of, "signaling entities" as used herein.

As used herein, "fastened to or adapted to be fastened", in the context of a species relative to another species or to a surface of an article, means that the species is chemically or biochemically linked via covalent attachment, attachment via specific biological binding (e.g., biotin/streptavidin), coordinative bonding such as chelate/metal binding, or the like. For example, "fastened" in this context includes multiple chemical linkages, multiple chemical/biological linkages, etc., including, but not limited to, a binding species such as a peptide synthesized on a polystyrene bead, a binding species specifically biologically coupled to an antibody which is bound to a protein such as protein A, which is covalently attached to a bead, a binding species that forms a part (via genetic engineering) of a molecule such as GST or Phage, which in turn is specifically biologically bound to a binding partner covalently fastened to a surface (e.g., glutathione in the case of GST), etc. As another example, a moiety covalently linked to a thiol is adapted to be fastened to a gold surface since thiols bind gold covalently. Similarly, a species carrying a metal binding tag is adapted to be fastened to a surface that carries a molecule covalently attached to the surface (such as thiol/gold binding) which molecule also presents a chelate coordinating a metal. A species also is adapted to be fastened to a surface if a surface carries a particular nucleotide sequence, and the species includes a complementary nucleotide sequence.

"Covalently fastened" means fastened via nothing other than one or more covalent bonds. E.g. a species that is covalently coupled, via EDC/NHS chemistry, to a carboxylate-presenting alkyl thiol which is in turn fastened to a gold surface, is covalently fastened to that surface.

As used herein, a component that is "immobilized relative to" another component either is fastened to the other component or is indirectly fastened to the other component, e.g., by being fastened to a third component to which the other component also is fastened, or otherwise is translationally associated with the other component. For example, a signaling entity is immobilized with respect to a binding species if the signaling entity is fastened to the binding species, is fastened to a colloid particle to which the binding species is fastened, is fastened to a dendrimer or polymer to which the binding species is fastened, etc. A colloid particle is immobilized relative to another colloid particle if a species fastened to the surface of the first colloid particle attaches to an entity, and a species on the surface of the second colloid particle attaches to the same entity, where the entity can be a single entity, a complex entity of multiple species, a cell, another particle, etc. All entities that can be fastened or adapted to be fastened to other entities of the invention also can be immobilized or adapted to be immobilized to the other entities, and vice versa.

"Specifically fastened" or "adapted to be specifically fastened" means a species is chemically or biochemically linked to another specimen or to a surface as described above with respect to the definition of "fastened to or adapted to be fastened", but excluding all non-specific binding.

"Non-specific binding", as used herein, is given its ordinary meaning in the field of biochemistry.

"Colloids", as used herein, means very small, self-suspendable particles including inorganic, polymeric, and metal particles. Typically, colloid particles are of less than 250 nm cross section in any dimension, more typically less than 100 nm cross section in any dimension. As used herein this term includes the definition commonly used in the field of biochemistry, and it typically means gold colloid particles

A "moiety that can coordinate a metal", a used herein, means any molecule that can occupy at least two coordination sites on a metal atom, such as a metal binding tag or a chelate.

"Diverse biological species" means different animals, such as mouse and hamster, mouse and goat, etc.

The term "sample" refers to any cell, tissue, or fluid from a biological source (a "biological sample"), or any other medium, biological or non-biological, that can advantageously be evaluated in accordance with the invention including, but not limited to, a biological sample drawn from a human patient, a sample drawn from an animal, a sample drawn from food designed for human consumption, a sample including food designed for animal consumption such as livestock feed, milk, an organ donation sample, a sample of blood destined for a blood supply, a sample from a water supply, or the like. One example of a sample is a sample drawn from a human or animal to whom a candidate drug has been given to determine the efficacy of the drug.

A "sample suspected of containing" a particular component means a sample with respect to which the content of the component is unknown. For example, a fluid sample from a human suspected of having a disease, such as a neurodegenerative disease or a non-neurodegenerative disease, but not known to have the disease, defines a sample suspected of containing neurodegenerative disease aggregate-forming species. "Sample" in this context includes naturally-occurring samples, such as physiological samples from humans or other animals, samples from food, livestock feed, etc., as well as "structurally predetermined samples", which are defined herein to mean samples, the chemical or biological sequence or structure of which is a predetermined structure used in an assay designed to test whether the structure is associated with a particular process such as a neurodegenerative disease. For example, a "structurally predetermined sample" includes a peptide sequence, random peptide sequence in a phage display library, and the like. Typical samples taken from humans or other animals include cells, blood, urine, ocular fluid, saliva, cerebro-spinal fluid, fluid or other samples from tonsils, lymph nodes, needle biopsies, etc.

As used herein, a "metal binding tag" refers to a group of molecules that can become fastened to a metal that is coordinated by a chelate. Suitable groups of such molecules include amino acid sequences, typically from about 2 to about 10 amino acid residues. These include, but are not limited to, histidines and cysteines ("polyamino acid tags"). Such binding tags, when they include histidine, can be referred to as a "poly-histidine tract" or "histidine tag" or "HIS-tag", and can be present at either the amino- or carboxy-terminus, or at any exposed region, of a peptide or protein or nucleic acid. A poly-histidine tract of six to ten residues is preferred for use in the invention. The poly-histidine tract is also defined functionally as being a number of consecutive histidine residues added to a protein of interest which allows the affinity purification of the resulting protein on a metal chelate column, or the identification of a protein terminus through the interaction with another molecule (e.g. an antibody reactive with the HIS-tag).

"Affinity tag" is given its ordinary meaning in the art. Affinity tags include, for example, metal binding tags, GST (in GST/glutathione binding clip), and streptavidin (in biotin/streptavidin binding). At various locations herein specific affinity tags are described in connection with binding interactions. It is to be understood that the invention involves, in any embodiment employing an affinity tag, a series of individual embodiments each involving selection of any of the affinity tags described herein.

"Molecular wires" as used herein, means wires that enhance the ability for a fluid encountering a SAM-coated electrode to communicate electrically with the electrode. This includes conductive molecules or, as mentioned above and exemplified more fully below, molecules that can cause defects in the SAM allowing communication with the electrode. A non-limiting list of additional molecular wires includes 2-mercaptopyridine, 2-mercaptobenzothiazole, dithiothreitol, 1, 2-benzenedithiol, 1, 2-benzenedimethanethiol, benzene-ethanethiol, and 2-mercaptoethylether. Conductivity of a monolayer can also be enhanced by the addition of molecules that promote conductivity in the plane of the electrode. Conducting SAMs can be composed of, but are not limited to: 1) poly (ethynylphenyl) chains terminated with a sulfur; 2) an alkyl thiol terminated with a benzene ring; 3) an alkyl thiol terminated with a DNA base; 4) any sulfur terminated species that packs poorly into a monolayer; 5) all of the above plus or minus alkyl thiol spacer molecules terminated with either ethylene glycol units or methyl groups to inhibit non specific adsorption. Thiols are described because of their affinity for gold in ready formation of a SAM. Other molecules can be substituted for thiols as known in the art from U.S. Patent No. 5,620,820, and other references. Molecular wires typically, because of their bulk or other conformation, creates defects in an otherwise relatively tightly-packed SAM to prevent the SAM from tightly sealing the surface against fluids to which it is exposed. The molecular wire causes disruption of the tightly-packed self-assembled structure, thereby defining defects that allow fluid to which the surface is exposed to communicate electrically with the surface. In this context, the fluid communicates electrically with the surface by contacting the surface or coming in close enough proximity to the surface that electronic communication via tunneling or the like, can occur.

The term "biological binding" refers to the interaction between a corresponding pair of molecules that exhibit mutual affinity or binding capacity, typically specific or non-specific binding or interaction, including biochemical, physiological, and/or pharmaceutical interactions. Biological binding defines a type of interaction that occurs between pairs of molecules including proteins, nucleic acids, glycoproteins, carbohydrates, hormones and the like. Specific examples include antibody/antigen, antibody/hapten, enzyme/substrate, enzyme/inhibitor, enzyme/cofactor, binding protein/substrate, carrier protein/substrate, lectin/carbohydrate, receptor/hormone, receptor/effector, complementary strands of nucleic acid, protein/nucleic acid repressor/inducer, ligand/cell surface receptor, virus/ligand, etc.

The term "binding partner" refers to a molecule that can undergo binding with a particular molecule. Biological binding partners are examples. For example, Protein A is a binding partner of the biological molecule IgG, and vice versa..

The term "determining" refers to quantitative or qualitative analysis of a species via, for example, spectroscopy, ellipsometry, piezoelectric measurement, immunoassay, electrochemical measurement, and the like. "Determining" also means detecting or quantifying interaction between species, e.g. detection of binding between two species.

The term "self-assembled monolayer" (SAM) refers to a relatively ordered assembly of molecules spontaneously chemisorbed on a surface, in which the molecules are oriented approximately parallel to each other and roughly perpendicular to the surface. Each of the molecules includes a functional group that adheres to the surface, and a portion that interacts with neighboring molecules in the monolayer to form the relatively ordered array. See Laibinis, P. E.; Hickman, J.; Wrighton, M. S.; Whitesides, G. M. Science 245, 845 (1989), Bain, C.; Evall, J.; Whitesides, G. M. J. Am. Chem. Soc. 111, 7155-7164 (1989), Bain, C.; Whitesides, G. M. J. Am. Chem. Soc. 111, 7164-7175 (1989), each of which is incorporated herein by reference.

The term "self-assembled mixed monolayer" refers to a heterogeneous self-assembled monolayer, that is, one made up of a relatively ordered assembly of at least two different molecules.

In one aspect, the present invention contemplates interaction between chemical or biological agents for analysis, drug screening, or the like. The invention includes but is not limited to analyzing and/or inhibiting ligand interactions, including but not limited to ligands on intact cells (growing on an electrode, or in solution or in suspension). The present invention contemplates a variety of embodiments, including the use of drug candidates, known or putative ligands, and small molecule drug libraries.

In one embodiment, cells are grown on electrodes that may or may not be derivatized with self-assembled monolayers (SAMs). Putative ligands (e.g. for a particular cell-surface receptor) are immobilized on a solid support (e.g. gold colloids) along with signaling elements (e.g. electroactive complexes). These derivatized solid supports are incubated with the cells immobilized on a sensing electrode (e.g. metal support). The interaction between the target receptor and the ligand on the solid support (e.g. colloid-bound ligand) tethers the co-immobilized signaling elements near the sensing electrode. While not limited to any particular mechanisms, it is believed that, as a potential is applied to the electrode, the nearby redox-active metal complexes go through their characteristic oxidation potential and eject electrons. When an oscillating component is added on top of the voltage ramp, many electrons are ejected by each metal complex and can be detected as current output. A form of this sort of electrochemical analysis is called alternating current voltammetry (ACV). As would be known by those of ordinary skill in the art, as used herein, "cell-surface receptor" is a generic term encompassing also cell surface proteins.

Certain embodiments of the invention make use of techniques for forming self-assembled monolayers on surfaces, and articles having surfaces coated with SAMs. In one set of preferred embodiments, SAMs formed completely of synthetic molecules completely cover a surface or a region of a surface, e.g. completely cover the surface of a colloid particle. "Completely cover" in this context, means that there is no portion of the surface or region that directly contacts a protein, antibody, or other species that prevents complete, direct coverage with the SAM. I.e. the surface or region includes, across its entirety, a SAM consisting completely of non-naturally-occurring molecules (i.e. synthetic molecules). The SAM can be made up completely of SAM-forming species that form close-packed SAMs at surfaces, these species in combination with molecular wires or other species able to promote electronic communication through the SAM (including defect-promoting species able to participate in a SAM), other species able to participate in a SAM, and any combination of these. Preferably, all of the species that participate in the SAM include a functionality that binds, optionally covalently, to the surface. In some embodiments a self-assembled monolayer is formed on a gold colloid. A self-assembled monolayer, whether formed on a colloid or on another surface, can be comprised of a mixture of thiol species (when gold is the surface) that include tri-ethylene glycol-terminated thiols to resist non-specific adsorption and thiols terminating in a binding partner of an affinity tag, e.g. terminating in a chelate that can coordinate a metal such as nitrilo tri-acetic acid which, when in complex with nickel atoms, capture histidine-tagged binding species. In a preferred embodiment the binding species is a beta-amyloid peptide which can readily self-aggregate. The present invention provides a method for rigorously controlling the concentration of the histidine-tagged peptides presented on the colloid surface. Without this rigorous control over peptide density on each colloid particle, co-immobilized peptides would readily aggregate with each other to form micro-hydrophobic-domains that would catalyze colloid-colloid aggregation in the absence of aggregate-forming species present in a sample. This is an advantage of the present invention, over existing colloid agglutination assays.

The methods described in the present invention produce self-assembled monolayers on colloids that resist non-specific adsorption without protein blocking steps, such as blocking with BSA. The methods described herein also produce derivatized colloids that are stable in biologically relevant fluids and do not require detergents (for stability; maintaining colloids in suspension), which interfere with binding reactions. This allows sensitive binding assays to be performed in solution. This abrogates the need for having binding partners adhered to adsorbent surfaces, as is common for existing colloid agglutination assays. As is discussed below, detergent can advantageously be used for SAM formation on colloids. In this case, detergent can be and preferably is removed after SAM formation and is no longer present on the colloid, in the SAM, or elsewhere during binding interactions or other use of the colloids.

It is not intended that the present invention be limited to embodiments wherein the cells are bound directly to a the metal support (e.g., electrodes). Embodiments are contemplated wherein the cells are indirectly bound through the interaction of ligands attached to the metal support. That is to say, cells can also be recruited to a surface by coating that surface with molecules that directly or indirectly bind to cells, by specific or nonspecific interactions. For example, methyl-terminated self-assembled monolayers (SAMs) bind collagen non-specifically, which in turn binds cells non-specifically. Alternatively, peptides that contain an argenine, glycine, aspartate (RGD) motifs, mimic vitronectin and bind specific cell types, like endothelial cells. Similarly, polylysine, positive charge, Kringle domains, integrins, and peptide, or molecular mimics of the same, can be bound to surfaces for the attachment of cells. These ligands can be displayed on a surface by incorporation into SAMs. The ligands themselves need not be directly incorporated into the SAM. SAMs that display a binding partner for an affinity tag attached to the ligand may be used. For example, peptides modified with a histidine tag can be easily attached to SAMs that contain a nitrilo tri-acetic acid (NTA) - nickel thiol. Alternatively, glutathione S-transferase fusion proteins can be attached to a SAM that incorporates glutathione or a derivative thereof. One advantage of using a metal electrode is that many known functional groups can be provided at the terminus of molecules that will form a SAM on the surface.

Cells in solution, for example, can be attracted to a detecting electrode by electrophoresis. Specifically, cell-derived molecules can be bound to a ligand(s) that are attached to a colloid that also displays electro-active compounds such as ferrocene derivatives to aid in the detection of the bound complex. However, the detection element used can be any charged, electro-active species or fluorescent tag that can be easily detected or the colloid itself.

Signaling entities are used in a variety of assays and arrangements of the invention. It is to be understood that any of a variety of signaling entities can be selected in each case, including a dye, pigment, electroactive molecule, fluorescent moiety, up-regulating phosphor, enzyme-linked signaling moiety including horse radish peroxidase and alkaline phosphatase, chemiluminescent moiety, electrochemiluminescent moiety, etc. See for example, Knight, "Trends in Analytical Chemistry", vol. 18, 1999, pg. 47; Knight, et al., Analyst, vol. 119, 1994, page 879; Stults, et al., "Use of Recombinant Biotinylated Aequorin in Microtiter and Membrane-Based Assays: Purification of Recombinant Apoaequorin from escheria coli", Biochemistry, 1992, 31, 1433; Mengeling, et al., "A Microplate Assay for Analysis for Solution-Phase Glycosyltransferase Reactions: Determination of Kinetic Constants", Analytical Biochemistry, 119, 286, (1991). A variety of signaling entities can be immobilized relative to surfaces of articles such as beads or colloid particles, if desired. Signaling entities presented on any of these can be fluorescent molecules. Fluorescent-conjugated antibodies and other fluorescent fusion proteins, including green fluorescent proteins, are widely used in biomedical research and testing. These fluorescent proteins and molecules can easily be attached to gold colloids that also present putative binding partners either through affinity tags, EDC/NHS chemistry or by binding to a His-tagged protein A or G presented on NTA-SAM-coated colloids according to the invention. Signaling entities such as fluorescent moieties also can be co-immobilized on a colloid via a biotin terminated ligand, or may be fastened via a chelate/metal/metal binding tag linkage. A fluorescent moiety may also be fastened by attaching it to an antibody and using a chelate/metal/metal binding tag with His-protein G to bind the antibody. The moieties can then be directly detected.

The ligand derivatized colloids can be tethered near an electrode that may or may not be modified with a self-assembled monolayer (SAM) and can detect the bound cells by perturbations in the electrode current induced by the binding. Cell-derived proteins can also be bound to a ligand(s) that is attached to a magnetic bead. Alternatively, cell-derived proteins can also be bound to a ligand(s) on an electroactive dendrimer. Additionally, cell-derived proteins can be bound with a ligand(s) that is attached to a polymer that also displays electro-active or redox-active complexes. Cell-derived proteins can also be bound with a ligand(s) that is attached to a conducting polymer that also displays electro-active or redox-active complexes.

In an alternative scheme, the described system can make use of two different ligands. One ligand, which binds a to a constitutively expressed cell surface receptor, can be immobilized on a magnetic bead and used to recruit the cell to a sensing electrode. A second ligand which recognizes the cell surface receptor of interest is attached to a signaling colloid.

In another alternative scheme, the described system can make use of two distinguishable electro-active complexes, such as two ferrocene derivatives that oxidize at different potentials. The first ferrocene derivative can be directly or indirectly attached to a ligand for the cell-surface receptor of interest. A second ferrocene derivative is directly or indirectly attached to a second ligand that binds to a constitutively expressed cell surface receptor. In this way, the ratio of the two signals can be used to calibrate the level of induction of a cell-derived molecule (e.g. protein) that is induced by a physiological, environmental, or disease-associated change in expression. This comparison of expression ratios can also be done by having both ligands carry the same metal complex but incubating separate aliquots of the sample on separate electrode pads.

The present invention contemplates the attachment of ligands (including but not limited to putative drug candidates) to a surface that can be particle-like and interact them with cell-derived proteins, e.g. cell surface proteins, that can be attached to supports or left intact on cells in an effort to identify binding partners, determine their presence or absence, and quantify their levels. Specifically, intact cells that present cell surface receptors can be used as the first binding partner. Known or putative ligands attached directly or indirectly to signaling entities act as the second binding partner. Alternatively, cell-derived proteins can be bound to a surface, and their ligand or binding partner attached to a particle that has signaling capability, such as a colloid or derivatized colloid. Drug candidates can be added to facilitate drug screening through disruption of the interaction. This method is also very useful in diagnostics. A protein or antibody can be immobilized on a surface, and a sample suspected of containing a binding partner to this protein can be incubated with the sample to facilitate binding, and the rest of the sample can be washed away. A particle bearing a ligand to the binding partner and a signaling capability can be added. If the sample contains the binding partner, the particle will be indirectly bound to the surface to give a signal. The surfaces can be recruitable particles. Attachments can be direct or indirect, i.e., attachments can involve two entities becoming immobilized with respect to each other. Particles can display immobilized antibodies, e.g., histidine-tagged Protein G to which antibodies bind, and the antibodies can recognize a common ligand, or can contain proteins that recognize each other.

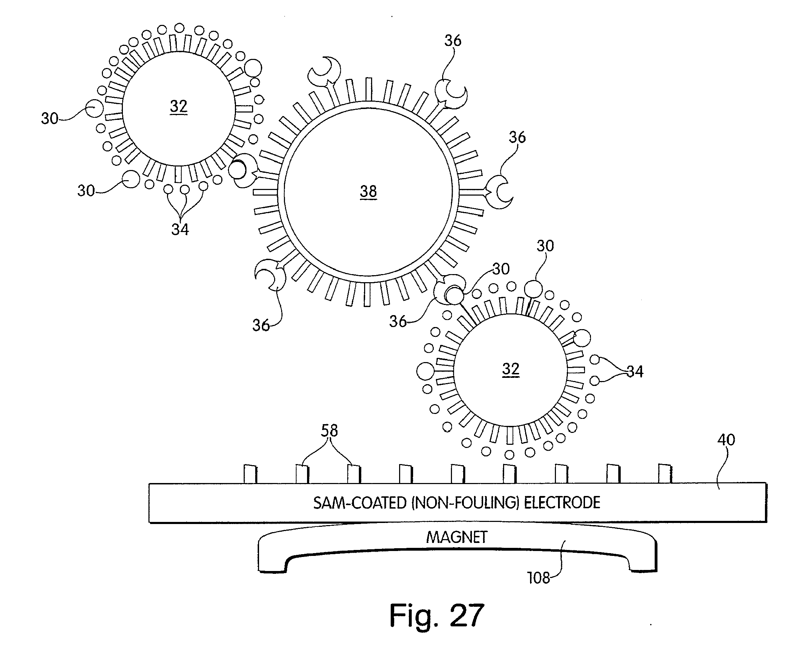

One general technique of the invention involves using magnetic beads to recruit an electronic or electrochemical signaling entity to the surface of an electrode indicating capture of a binding moiety (a biological or chemical agent) by a binding partner. Referring to Figure 27, a first species 30 is attached to a colloidal particle 32 that also has a signaling capability such as the metallocene ferrocene, 34. A second species 36, which is suspected of being a binding partner of 30 is attached to a magnetic particle 38 that cannot signal, but can be magnetically attracted to an electrode 40. In one particularly useful technique species 30 and a second species 36 are proteins, thus the invention finds particular use in the field of proteomics. The technique is a example of an aspect of the invention involving allowing a colloid particle 32 the ability to fasten to non-colloidal structure, magnetic bead, 38. Determining fastening of the colloid particle to the non-colloidal structure is carried out by drawing the magnetic bead to the electrode and determining whether the colloid particle is also proximate the electrode, or unattached. Specifically, the two components are incubated together in solution and resultant complexes are magnetically attracted to the electrode. Electrodes are then analyzed by Alternating Current Voltammetry (ACV). (Laviron E: J Electro Anal Chem., 1979, 105: 35). Current is plotted, in real time, as a function of voltage. If the electronic or electrochemical signaling moiety on the first component is brought very close to the electrode, a distinctive current peak will occur at a characteristic potential. If the two proteins interact with each other, then when the magnetic particle is attracted to the sensing electrode, it will also carry the colloidal particle, with the signaling capability, with it. Because the first component is a small gold colloid, it will remain in suspension unless it is specifically recruited to the sensing electrode via an interaction with the first species on the colloidal particle and the second species attached to the magnetic particle. An electronic or electrochemical signal will only be generated if the signaling particle has been connected to the magnetic particle through an interaction between the first species and the second species. In this embodiment, the colloid particle comprises an auxiliary signaling entity, exemplified by ferrocene. In other embodiments described below, the colloid particle is itself a signaling entity and no auxiliary entity is required.

This strategy can be used to rapidly screen for protein-protein interactions in array format. When searching for potential therapeutic targets, researchers often need to identify a binding partner for a particular protein. The known protein can be attached to a magnetic bead. This can be accomplished by direct chemical coupling of the known protein to commercially available magnetic beads that present chemical functional groups, by attaching to a protein A-coated magnetic bead via a cognate antibody, or by attaching a histidine-tagged version of the known protein to a gold-coated magnetic bead that has been derivatized with an NTA-SAM (see, for example, U.S. Patent No. 5,620,850, incorporated herein by reference). Potential binding partners can be expressed as histidine-tagged proteins then separately attached to NTA-SAM-coated colloids that also present a ferrocene moiety. Each well of a multi-well array, interfaced with an electrode array, will contain the known protein on a magnetic bead. Distinct putative binding partners attached to signaling colloids can be separately added to each well. Resultant complexes can then be magnetically attracted to the electrodes to determined which of the candidate proteins, bound the known protein. The known species need not necessarily be a protein. When binding partners have been identified, drug candidates can be added to the assay to disrupt the interaction.

Alternatively, biological or chemical agent 30 is a drug candidate and its binding partner (potential binding partner) 36 is a target of a drug candidate or vice versa. Specifically, drug candidates can be attached to the magnetic particles and the known target species attached to signaling colloids, to identify drug candidates that bind to a medically relevant target species. Or, species 30 and 36 can be a target and ligand to the target, respectively, and the technique can involve bringing the colloid 32 and magnetic bead 38 into proximity to allow fastening of 30 to 36 in the presence of a candidate drug for interruption of binding of the ligand to the target. As will be seen below, this technique can be applied to interaction of a colloid with a variety of non-colloidal structures.

Advantages of this technology over existing methods such as ELISA, fluorescent labeling and SPR include: In this system, there is no need for protein labeling; the protein is attached to a labeled component. Gold colloids can be pre-labeled with both: a) a signaling moiety; and b) a functional group for protein attachment. Self-assembled monolayers that present both NTA/Ni²⁺, to capture histidine-tagged proteins, and a ferrocene derivative, for electronic or electrochemical signaling, can be formed on the colloids. SAMs that incorporate carboxylic acid groups, for the chemical coupling (standard EDC/NHS chemistry) of unmodified proteins, can also be used. The technology is modular. Virtually any biological species can be co-immobilized on colloids with a signaling entity. The technology enables cost-effective multiplexing as it can readily be multiplexed on microelectrode arrays.

Liposomes can also incorporate the first binding partner 30. As above, the signaling itself can be incorporated into the liposome or lipids can be incorporated into the liposomes in order to capture His-tagged soluble receptors after liposome formation. The liposomes can contain photoactivatable groups that allow for UV-activated cross linking after liposome formation in order to give increased stability.

Identification of drug candidates can also be accomplished by using a competitive inhibition assay. Specifically, a drug candidate free in solution can be separately incubated with the composition. Competitive inhibition to the target cell-derived protein occurring by drug binding to receptor or ligand can be observed as a time or dosage dependent loss of detection signal.

Doing this requires a method of directly or indirectly attaching a signaling moiety to a known ligand and then provided a method of attaching or recruiting the complex binding partners to a sensing surface. The former is described above while the latter is described below.

The sensing surface used can take various forms. However, for illustration purposes, methods for recruiting the binding partner complex are described that use an electrode that is modified with a conducting SAM. A conducting SAM is a layer of molecules attached to a metal surface that allows the conduction of electrons at a rate that is higher than a metal uniformly coated with an insulating species such as saturated alkyl thiolates. A preferred pathway for electron conduction can be provided by a monolayer into which molecular wires (polymers of aromatic ring compounds) have been incorporated. A variety of molecules can be used for this purpose, including but not limited to poly (ethynylphenyl thiol) (i.e. C₁₆H₁₀S), referred to herein as MF1.:

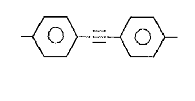

"Molecular wires" as used herein, means wires that enhance the ability for a fluid encountering a SAM-coded electrode to communicate electrically with the electrode. This includes conductive molecules or, as mentioned above and exemplified more fully below, molecules that can cause defects in the SAM allowing fluid contact with the electrode. A non-limiting list of additional molecular wires includes 2-mercaptopyridine, 2-mercaptobenzothiazole, dithiothreitol, 1, 2-benzenedithiol, 1, 2-benzenedimethanethiol, benzene-ethanethiol, and 2-mercaptoethylether. Conductivity of a monolayer can also be enhanced by the addition of molecules that promote conductivity in the plane of the electrode. Conducting SAMs can be composed of, but are not limited to: 1) poly (ethynylphenyl) chains terminated with a sulfur; 2) an alkyl thiol terminated with a benzene ring; 3) an alkyl thiol terminated with a DNA base; 4) any sulfur terminated species that packs poorly into a monolayer; 5) all of the above plus or minus alkyl thiol spacer molecules terminated with either ethylene glycol units or methyl groups to inhibit non specific adsorption. Thiols are described because of their affinity for gold in ready formation of a SAM. Other molecules can be substituted for thiols as known in the art from U.S. Patent No. 5,620,820, and other references.

When known ligands on signaling colloids interact with their cognate cell derived proteins, drug candidates can be added in solution and differential effects monitored. Cells can be recruited to an electrode coated with a conducting SAM that also presents molecules that are terminated with head groups that directly or indirectly bind to cells (e.g. methyl groups, poly K, positive charge, RGD sequences, Kringle motifs, integrins, and peptide mimics of the same). Alternatively differential interactions can be quantified by having cell surface receptors that are interacting with their cognate ligands presented on a conducting SAM that are linked to the SAM. In this situation, the electronic or electrochemical signal is generated after a ligand binds to a constitutively expressed receptor. Under flow force the electronic or electrochemical signal should be proportional to the number of cell surface receptors interacting with SAM-immobilized ligands. The rate of cell mobility as a function of flow rate indicates the density of receptors to cell adhesion molecules on the surface. Downstream of the interactive SAM is a charge reader (e.g. ammeter, charge counter) or an optical reader ( e.g. surface plasmon resonance detector, or fluorescence reader). In addition, these methods can also be used to screen for drugs that inhibit a cell phenotype known to be characteristic to invasiveness or metastasis without a priori knowledge of the specific receptor-adhesion molecule interactions involved. Screening for drugs that mimic a physiologically relevant interaction can also be accomplished using this system whether by adding a drug candidate free in solution and looking for a loss of detection signal, or by adding a known ligand free in solution with the drug candidate on the solid support and looking for the occurrence of a detection signal.

Cells are attracted to the sensing electrode due to the fact that they are negatively charged when a slight positive bias to the AC voltage ramp is applied. Adding additional negatively charged groups to the dendrimers or polymers to which the ligands are attached further facilitates recruitment to the sensing surface. Those of ordinary skill in the art can readily fasten a plurality of signaling entities to a dendrimer or polymer. Alternatively, attaching the recognition ligands via metal binding tags facilitates their binding (and consequently the binding of the associated cell) to alkyl thiol NTA. SAMs having metal containing compounds, such as ferrocene, on a gold coated magnetic bead allow the use of an electromagnetic field or a stationary magnet to recruit the complex to the sensing surface electrode. Also sensing electrodes that incorporate ligands, either directly or through a histindine tag, can be used to attract cells via specific interactions, such as with cell adhesion molecules or non specific interactions.

However, recruitment to the detection system can be performed also simply by using gravity if the complex sediments by gravity as a result of one of the binding partners being denser than the analysis solution. Mechanical mixing can be performed during the incubation stage to avoid premature sedimentation. Figs. 32A and 32B show ACV plots of an experiment demonstrating the use of gravity to recruit a signaling entity indicating a binding interaction to a surface, and a control. GST was bound to colloids via a metal binding tag/metal/chelate linkage (NTA-SAM). The colloids also presented ferrocene derivatives for electronic or electrochemical signaling. As a negative control, the colloids were presented in solution to a measuring electrode and the plot of Fig. 32A was derived. One set of colloids was exposed to polymeric beads (agarose) coated with glutathione. The colloids became fastened to the beads via glutathione/GST binding, and the beads settled onto the electrode by gravity. The ACV plot of Fig. 32B resulted, showing electroactive detection of ferrocene approximate the electrode.

In a variation that uses the electrode as a cell derived protein generator, a point on the electrode pad can be used to produce a voltage spike that lyses the cell. The contents of the cell are then incubated with signaling ligands and interactions are detected.

Additionally, cell binding molecules can be patterned in a given topology along the bottom of a flow channel using SAMs in order to line the channel with conducting molecules and enhance the detection abilities of the system. Thus, the SAM can present the binding moiety in a pattern that alternates cell binding capability with sensing capability (optical or electronic or electrochemical). As an example, stripes of methyl terminated alkyl thiols are alternated with conducting poly pyrrole groups in the SAM. The topology of the SAM can also be manipulated such that the cell lies in a furrow so that receptors along the sides of the cell can be assessed. Forming stripes of alternating functionality SAMs such as these can be carried out as described in U.S. Patent No. 5,512,131 and International Patent Publication No. WO 96/29629, both incorporated herein by reference. These complexes can be electronically or electrochemically detected. One detection method is an electrochemical technique called alternating current voltammetry (ACV). This detection method can be supplemented by the use of additional analysis techniques such as higher order harmonic analysis. These complexes may also be able to be detected by optical means such as SPR. In using the latter technique, large shifts in optical properties are caused by recruitment of the complexes to the sensing surface.

In one embodiment, one can detect and quantitate cell surface proteins as follows: Histidine-tagged ligands that recognize cell surface receptors are attached to colloids that bear SAMs presenting both NTA (to capture His-tagged proteins) and ferrocene moieties (for electronic or electrochemical signaling). These biospecific, electronic or electrochemical signaling colloids are then incubated with cells presenting target receptors. Cells are then allowed to sediment, adhere, or be attracted onto to a SAM-coated electrode and analyzed by ACV (Figure 3). A current peak, at the ferrocene moiety's characteristic oxidation potential, will result if ligands immobilized on signaling colloids bound to their cognate receptors on the cell surface. Antibodies that recognize the cell surface receptor can be attached to NTA-ferrocene bearing colloids that have first been bound with His-tagged protein A or G. Alternatively, an antibody can be attached directly to a colloid via a metal binding tag/metal/chelate linkage, where the metal binding tag is linked to the antibody. Techniques for linking a histidine tag to an antibody can be found in "Construction of the single-chain Fv from 196-14 antibody toward ovarian cancer-associated antigen CA125" Hashimoto, Y., Tanigawa, K., Nakashima, M., Sonoda, K., Ueda, T., Watanabe, T., and Imoto, T.: 1999, Biological and Pharmaceutical Bulletin, Vol 22: (10) 1068-1072.; "Human antibodies with sub-nanomolar affinities isolated from a large non-immunized phage display library", Vaughan, T.J., Williams, A.J., Pritchard, K., Osbourn, J.K., Pope, A.R., Earnshaw, J.C. et al. 1996, Nature Biotechnology Vol 14 (3) p. 267.; "Expression and purification of single chain anti-HBx antibody in E. coli" Zhou G, lui KD, Sun H.C., Chen Y.H., Tang Z.Y., and Schroder C.H., 1997, vol. 123(11-12) pgs 609-13.

As one example of a technique of the invention, with reference to Figure 4, an example of a useful technique involving fastening of a colloid particle to a cell is described. The tumor marker, MUC-1, is aberrantly expressed on neoplastic cells. The human tissue culture breast carcinoma cell line, MCF-7, available from the ATCC, over-expresses MUC-1. Antibody 50, DF3 or and DF3-p, available from the Dana-Farber Cancer Institute, is attached to electronic or electrochemical signaling colloids 52 (bearing NTA-SAMs 54) via a histidine-tagged protein G 56. Target cells 58 are incubated with the antibody-bearing signaling colloids 52, then electrophoresed to an electrode 40 coated with a SAM containing molecular wires 58 and analyzed by ACV. The SAM on electrode 40 includes molecular wires 58 admixed within more conventional, tight-packing SAM-forming species. For simplicity of illustration in all figures, only molecular wires 58 are shown schematically. A current peak results if the antibody-bearing signaling colloids are incubated with cells bearing MUC-1. Alternatively, the putative cognate ligand for MUC-1, I-CAM can be His-tagged and attached to signaling colloids that also bear NTA groups.

Another assay is shown in Figure 5. Drug libraries can be screened for their ability to disrupt specific interactions with cell surface proteins, such as the MUC-1/1-CAM interaction. I-CAM 64 is bound to signaling colloids 62 as described above, then incubated with cells 70 presenting MUC-1 (66) and control cells. Drug candidates 68 are added to the solution within which the cells and colloids are suspended, then the cells adhere to the electrode and analyzed by ACV. A loss of signal indicates an interaction with a drug candidate. Figure 6 illustrates how this scheme can readily be multiplexed to simultaneously screen thousands of drug candidates using arrays of disposable microwells 72 with interfacable arrays of microelectrodes 74. Each well contains an assay as shown in Figure 5 or another assay.

Referring now to Figure 7, an arrangement is shown in which for a gain of signal assay, or to screen for drugs to bind cell surface receptors 80 for which the cognate ligand is not known, small molecule drug libraries can be synthesized on, or covalently attached to, colloids or particles 78 that also bear electronic or electrochemical signaling moieties 34. Drug candidates 76 attached to signaling particles can be incubated with cells 82 presenting the receptor 80 of interest, or control cells 84. A drug-target interaction in this assay will result in a gain of signal (Figure 7).

The cell surface receptor, αVβ3, has been implicated in promoting angiogenesis through an interaction with a cell adhesion molecule vitronectin. Human umbilical veinous endothelial cells (HUVEC) that present αVβ3 cell surface receptors are commercially available from Clonetics. To screen for drugs that inhibit the action of αVβ3, His-tagged peptides that present RGD-containing sequences, derived from vitronectin, are attached to colloids that bear SAMs presenting both NTA groups and ferrocene moieties. The biospecific signaling colloids are then incubated with HUVEC cells and drug candidates. The HUVEC cells can be grown on the electrode. However, if the cells are in solution or suspension they can be electrophoresed or magnetically attracted to a sensing electrode and analyzed by ACV. A current peak occurs when biospecific colloids are incubated with HUVEC cells, rather than control cells. If a drug candidate interferes with the αVβ3-RGD sequence interaction, a loss of signal results. Viability of this assay as a screen for angiogenesis inhibitors was shown by disrupting the αVβ3-RGD interaction with the known angiognesis inhibitor endostatin. The assay can be conducted electronically, as discussed, or visually by growing the cells in a standard plastic dish, conducting the assay, and viewing the cells under 40-fold magnification.

Alternatively, drug candidates can be synthesized on, or attached to, beads, colloids or supports that also present electronic or electrochemical signaling moieties. These "particles" or surfaces are incubated with target cells, attracted to a sensing electrode and analyzed by ACV. The attractive field is then reversed and peptides containing RGD sequences are titrated into the solutions. The cells are again electrophoresed to the sensing electrode and re-analyzed. A loss of signal indicates that the drug-cell interaction is specific for the αVβ3 receptor and the IC₅₀ of the RGD peptide can be correlated to a binding affinity for the drug.

Alternatively, a 49 amino acid peptide, echistatin, that also binds to αVβ3, can be His-tagged to replace the RGD-containing peptide in the above-described assay.

Metallocenes are particularly useful as signaling entities for the following reasons. Various ferrocene derivatives and be selected to each oxidize at unique voltage between 100 mV to 800 mV. Each oxidation potential represents a unique label so that multiple cell surface targets can be simultaneously queried. If a biologically relevant interaction between a cell surface receptor and a colloid immobilized ligand occurs, the cell is decorated with electronic or electrochemical signaling particles and a current peak results. The magnitude of the current peak should be proportional to the number of cell surface receptors that were recognized by the signaling colloids.

Cell-surface molecules can be detected on cells in suspension or embedded in a tissue sample, as shown in Figure 8. Frozen tumor specimens 86 are cryo-sectioned and placed directly onto a flexible, semi-permeable membrane support 88 that has been derivatized with cell-binding groups 90 such as RGD-containing peptides or methyl-terminated groups. The specimen is then incubated with electronic or electrochemical signaling colloids 92 that also present ligands 94 for a cell surface receptor of interest. Unbound colloids are washed away after an incubation period. The support membrane is then placed in physical contact with a microelectrode array 96, having electrode dimensions comparable to cell size, and analyzed by ACV. Each sector of the tissue specimen is analyzed for protein content and expression level, then correlated with histopathology. This capability ensures the relevance of single cell analysis because it enables the researcher to identify protein patterns that are associated specifically with cancer cells and discard random aberrant protein expression. Cells in suspension can be similarly attached to the support membrane.

This technique can be used to identify cell-derived molecules, such as receptors or proteins, that are expressed differentially in healthy versus diseased tissue or cells. This differential expression can involve different levels of an expression in healthy versus diseased tissue or cells, and/or different patterns of expression on tissues or cells which can be readily identified. This technique facilitates diagnostic assays for determination of diseased states. For example, in connection with a patient suspected of having a particular disease, cells can be taken from the patient, specifically, cells that are associated with an indicator of the disease such as cells from a biopsy, blood sample, etc., and these cells can be analyzed versus healthy cells to determine expression levels or patterns indicative of disease. One can also use this system to screen for drugs that inhibit the upregulation of cell-derived proteins that are involved in various pathological conditions. Examples of two techniques for drug screening include: (1) administering a candidate drug to a patient suspected of or exhibiting symptoms of disease and monitoring a biological sample including cells of the patient as described above to determine efficacy of the drug in treatment of the disease; (2) taking a biological sample including cells from a patient suspected of having or having a disease and exposing the biological sample or components of the cells to candidate drugs, and monitoring expression level and/or pattern using the techniques described above. Once a binding partner (which can include a drug, antibody, or protein/peptide ligand for the cell derived protein) has been identified, the binding partner can be attached to a detection moiety to quantitate the expression level of the cell-derived protein in response to a disease state or a therapy. This can be any assay that tests for the indirect affects of drug candidates on the expression and translocation of cell-derived proteins both to the cell-surface and intracellular compartments.

The invention also provides the ability to visually investigate patterns of cell surface receptor expression on individual cell surfaces and/or on cells embedded in a tissue specimen. This can be indicative of the pattern of cell surface receptor expression which can be correlated to a disease state. These can also be used in diagnostics or drug screening methods. In a particular assay, colloid particles carrying ligands that bind to cell surface receptors are exposed to individual cells or embedded cells and the location of their binding with respect to individual cells can be determined visually, indicating the pattern of cell surface receptor expression. For example, MUC-1 is a cell surface receptor implicated in breast cancer. MUC-1 normally is expressed uniformly on surfaces of a variety of cell types. In transformed cells involved in a variety of cancers, the receptor is overexpressed and is concentrated at apical locations of the cell. This can be determined using the described technique. In drug screening, a culture of transformed cells can be provided and treated with drug candidates. The loss of the apical pattern expression is investigated. Visual identification, in this embodiment, can involve any technique described herein such as observation with the unaided human eye, microscopy, spectrophotometry, electron microscopy, fluorescence detection, etc.

In the technique involving electronic or electrochemical detection described above, the levels of expressed species can be compared between samples, including samples each involving an individual cell or other very small quantity, and patterns can be determined on larger samples including tissue samples. In connection with the visual detection embodiment described above, levels of expressed species can be determined as well as patterns of expressed species on both large samples and small samples including single-cell samples. Signaling entities useful in electronic or electrochemical detection include signaling entities described herein for electronic or electrochemical detection, including redox-active molecules such as ferrocenes. In connection with visual detection, any visual signaling entities described herein can be used including colloids, alone or carrying auxiliary signaling entities such as fluorescent or other visibly-identifiable entities. Multiple signaling entities can be used (i.e., multiple signaling per binding event). In connection with both electronic or electrochemical or visual signaling, different signaling entities can be used in connection with different assays. For example, a first ligand selected to target a first receptor or protein may be immobilized with respect to a first signaling entity while a second ligand, selected to target a second protein or receptor can be immobilized with respect to a second signaling entity. In electronic or electrochemical signaling the different signaling entities can include different redox potentials, the difference between which is distinguishable electronically, and in connection with visual identification different signaling entities can be different colors of emissive or absorptive entities. In such a case not only can expression level and pattern of proteins or receptors be determined but patterns can be differentiated in terms of location of expression of one receptor or protein versus another.

The biospecific colloids, described above, can also be used to facilitate *in vivo* imaging. The colloids used are gold, which is relatively inert. Gold colloids that have been derivatized to present an antibody or other ligand for a tumor marker can be taken internally or injected. A tumor mass presenting the cognate tumor marker will become covered with colloids, acting as a concentration device. The tumor can then be detected by imaging techniques, such as X-Ray and X-Ray computer tomography (CT), since the effective result can be a tumor mass enshrouded in metal. Individual colloids in solution would be invisible to detection. Tumors that have been so labeled with biospecific colloids can also be detected by MRI (magnetic resonance imaging). Imaging may be enhanced by incorporating paramagnetic metals and other contrast agents, such as Fe, Gd, or Cr. Contrast agents can be attached to the colloids by incorporating metal chelating thiols into the SAMs that are formed on the colloids. Alternatively, SAMs that only present a cognate ligand for the target protein expressed on the tumor, can be formed on particles composed of the contrast agent material. Colloids having an iron or magnetite core can be gold-coated then derivatized with biospecific SAMs. Alternatively, liposome-like particles can be formed from lipid chains terminated in chelating groups such that the metal contrast agents form the "core" and the biospecific ligands are exposed to solvent. Alternatively, the colloids can bear, in addition to the biospecific ligand, moieties that have a specific spectroscopic signature, which can be detected by energy absorption or scattering techniques, including Raman Spectroscopy. The imaging apparatus can be contained outside of the body or introduced into the body on scopes or optical fibers. Alternatively, colloids bearing moieties that transmit radio frequencies can generate a detectable signal if concentrated onto a tumor mass. The signal from individual colloids should be negligible or undetectable compared with the signal generated from a concentration of colloids bound to a tumor mass.

With reference to Figure 9, to determine whether two proteins interact with each other, one will bind the first protein 104 to a colloid 106 presenting both NTA 54 and ferrocene ligands 34 and bind the second protein 100 to a SAM-coated magnetic bead 102 that presents only the NTA ligand 54. The two particle types are mixed together then magnetically recruited to a sensing electrode 40 under which is a magnet 108. An electronic or electrochemical signal will only be generated if the magnetic bead were connected to the signaling colloid through the interaction of the two protein species. Non-histidine-tagged proteins can also be attached to signaling or magnetic (recruitable) particles by forming SAMs on them that incorporate carboxy-terminated thiols along with thiols terminated with an exposed group that is charged and that is not involved with the coupling chemistry (such as NTA thiols). Standard EDC/NHS coupling chemistry can then be used to attach any molecule that presents a primary amine. Because the electronic or electrochemical labeling step has been separated from the protein preparation step, the system can be efficiently multiplexed using universal electronic or electrochemical signaling colloids and magnetic particles. This facilitates the construction of protein interaction databases to help researchers decipher the molecular profiles of cancers.

Interacting protein partners can be incubated with small molecule drug libraries and drug leads identified by detecting a loss of signal. Alternatively, small molecule drug libraries can be purchased on magnetic beads to directly screen for drug candidates that bind to a known, target protein.

The basic technology described herein can also be used to screen for drugs to modulate enzyme activity. Certain enzymes have been identified as being critical to the progression diseases. In some cases a molecule must be cleaved at a specific site for it to become active and one would like to screen for drugs that inhibit the activity of the enzyme that does this. The technology described herein can be used as described below for this purpose:

Useful in carrying out this technique is the following: Edelstein and Distefano have reported that farnesyl pyrophosphate groups modified with photoactivatable cross-linking moieties can be added to peptide motifs derived from RAS by yeast FPT (Edelstein R. and Distefano M. (1997). Photoaffinity labeling of yeast farnesyl protein transferase and enzymatic synthesis of a RAS protein incorporating a photoactive isoprenoid. Biochem. and Biophys. Res. Comm. 235, 377-382). Their objective was to use this assay to show that farnesyl derivatives were recognized by FPT, by cross-linking the two. This finding is consistent with the idea that farnesyl or garenyl moieties can be modified with biotin at the end distal from the pyrophosphates without interfering with enzyme activity or specificity.

With reference to Figure 10, an enzyme cleavage site (ECS) 110 can be attached at one end 112 to a molecule 122 for which there is a known binding partner (BP1). At the other end 114, it can be attached to a molecule 116 that can incorporate into a SAM (HS-R-X), where S is sulfur, R is an molecular species that can incorporate into a SAM, and X is a linker. The resultant molecule can be incorporated into SAMs on colloids 116 along with groups 118 that can deliver an electronic signal (HS-R-XM), where M is a redox-active metal or transition metal (or other group capable of transducing an electronic or electrochemical signal when brought close to an electrode). Magnetic beads or particles 120 can be derivatized to present a molecule 124 (e.g., streptavidin) (BP2) that binds to BP1 122 either directly or indirectly through a simultaneous binding to a mutual target molecule (BP3).

For example, electronic or electrochemical signaling colloids 116 can be derivatized with thiols bearing enzyme cleavage sites (ECS) and terminated with biotin. Magnetic beads can be derivatized with streptavidin. Magnetic beads and signaling colloids can be incubated with the enzyme of interest and drug candidates. If a drug candidate that can moderate, or inhibit the action of the enzyme, then the site (of e.g., a protein or peptide) would not be cleaved, and the signaling colloid would be attached to the magnetic bead through the biotin/streptavidin interaction. When the complexes are magnetically attracted to a sensing electrode, a current peak would result, making the assay a gain of signal detection assay. That is, a protein can be adapted for linkage both to colloid 116 and bead 120 via a SAM-forming species at one end and a binding partner 122 linkable to an immobilized binding partner 124 on bead 120, and cleavage, or moderation thereof, monitored. The protein can be linked to the colloid and bead in a variety of ways including that described or, in addition, the colloid can present a chelate coordinating a metal, the protein can be provided with a metal binding tag. The invention is even more generalized, in that any entity adapted for linkage both to the colloid particle 116 and any non-colloidal structure, such as bead 120, can be presented to the colloid and the non-colloidal structure and allowed to link thereto, in the presence both of an enzyme having the ability to cleave the entity and a candidate drug for moderation of activity of the enzyme. The non-colloidal structure can be a bead as illustrated, or an electrode itself.

BP1 can be the same as BP2 and/or BP3. For example, the thiol that bears the ECS and BP1 (biotin) can bind to free streptavidin (BP3) then to biotin (BP2) on the magnetic beads. Alternatively, one may seek to inhibit or accelerate an enzyme that adds a molecular group to a target molecule, i.e., one can expose a colloid particle and a non-colloidal structure to a substrate for an enzyme adapted for linkage to the non-colloidal structure, a molecular species linkable to the substrate via enzyme activity adapted for linkage to the particle and an enzyme for the substrate. This can be carried out further in the presence of a candidate drug for moderation of the enzyme, and the non-colloidal structure can be a magnetic bead with the colloid particle carrying an immobilized electroactive entity. Alternatively, the non-colloidal structure can be an electrode surface.

Specifically, with reference to Figure 11, in such a case, electronic or electrochemical signaling colloids 126 are derivatized with the molecule 128 via chelate/metal/metal binding tag linkage, to which the group is to be added. The piece to be added, the addition molecule 130, is terminated with a first binding partner (BP1) 132 (can be biotin). The magnetic bead 12 presents a binding partner of BP1, (BP2) 134 (can be streptavidin). Colloids, magnetic beads, addition molecules, the enzyme of interest and drug candidates are incubated then attracted to an electrode for analysis. A loss of signal, compared to controls, indicates that the drug candidate inhibited the enzyme's activity while a gain of signal indicates a drug enhanced the enzyme's activity.

A wide variety of SAMs can be used in accordance with the invention, on a wide variety of surfaces, to present desired species such as binding partners, signaling entities, and the like at a surface of an article such as an electrode, colloid particle, or the like. Those of ordinary skill in the art can select from among a wide variety of surfaces, functional groups, spacer moieties, etc. An exemplary description can be found in U.S. Patent No. 5,620,850. This U.S. Patent also describes a variety of metal binding tags that can be used, including nitrilotriacetic acid, 2,2'-bis(salicylideneamino)-6,6'-demethyldiphenyl, and 1,8-bis(a-pyridyl)-3,6-dithiaoctane, or the like.

A variety of non-colloidal structures comprising beads are described above. The beads can comprise polymeric material, agarose, tentagel, and/or magnetic material. Polystyrene beads are quite useful. The function and advantage of these and other embodiments of the present invention will be more fully understood from the examples below. The following examples are intended to illustrate the benefits of the present invention, but do not exemplify the full scope of the invention.

The following examples and experiments illustrate particular embodiments of the present invention and are not to be construed as limiting the invention to any particular embodiment.

In some of the embodiments described below, the examples involve SAM formation, collagen coating, cell growth, colloid formation, and Alternating Current Voltammetry (ACV). For SAM formation, glass microscope slides were sputtered with a layer of Ti followed by a layer of Au. Each electrode was incubated at RT for 0.5 hours with 300 uL of a DMF solution that contained 10% methyl-terminated thiol (HS-(CH2)15 CH3), 40% tri-ethylene glycol-terminated thiol, HS(CH₂)₁₁(CH₂CH₂)₃OH, (formula) and 50% MF-1. 2 ml of 400 uM tri-ethylene glycol-terminated thiol were then added to a scintillation vial containing the chip and the vial was heat cycled in a water bath as follows: 2 minutes @ 55°C; 2 minutes @ 37°C; 1 minute @ 55°C; 2 minutes @ 37°C then RT for 10 min. Electrodes were then dipped in EtOH, then sterile PBS to rinse. They were then placed under the LTV germicidal lights in a biosafety cabinet for I hour to ensure sterility.

For collagen coating, a 200 uL droplet of 0.005 mg/ml collagen in PBS was added to each electrode and incubated at 4°C for 2 hours.

For cell growth, the electrodes were placed in a cell growth flask and a solution of growth media and human endothelial cells (HUVECs), presenting a particular cell surface receptor, αVβ3, was added. The electrodes and cell containing solution were incubated at 37 °C in a C0₂ incubator for 24 hours. Visual inspection with 100X magnification showed that the cells had adhered to the electrodes and showed web-like spreading which is a growth phenotype.

For colloid preparation, 1.5 ml of commercially available gold colloid (Auro Dye) were pelleted by centrifugation in a microfuge on high for 10 minutes. The pellet was resuspended in 100 uL of the storage buffer (sodium citrate and tween-20). 100 uL of a dimethyl formamide (DMF) solution containing 90 uM nitrilo tri-acetic acid (NTA)-thiol, 90 uM ferrocene-thiol, and 500 uM carboxy-terminated thiol. Following a 3-hour incubation in the thiol solution, the colloids were pelleted and the supernatant discarded. They were then incubated in 100 uL of 400 uM tri-ethylene glycol-terminated thiol in DMF for 2 minutes at 55 °C, 2 minutes at 37 °C, 1 minute at 55 °C, 2 minutes at 37 °C, then room temperature for 10 minutes. The colloids were then pelleted and 100 ul of phosphate buffered saline (PBS) were added. The colloids were then diluted 1:1 with 180 uM NiS04 in the colloid storage buffer. 100 uL of a His-tagged peptide at 100uM in PB S were added to 100uL of NTA-Ni(II) presenting colloids and incubated for 0.5 hours. To get rid of free, unattached peptide, the colloids were then pelleted and the supernatant discarded. The colloid pellet was then resuspended in 100uL PBS. Colloids were bound with either: a) a peptide designed to bind to the αVβ3 receptor, HHHHHH(S₄G₁)₃GRGDSGRGDS; or b) an irrelevant peptide, HHHHHH-Glutathione S-Transferase (GST). Peptides containing an RGD motif have been shown to bind to the αVβ3 receptor on endothelial cells. It is thought that RGD motifs in vitronectin (the natural ligand for αVβ3) are responsible for the interaction.

ACV Analysis was performed using a CH Instruments electrochemical analyzer. A three-electrode system was used. A silver vs. silver chloride reference electrode was used with a platinum auxiliary electrode. The derivatized gold-coated chip was used as the working electrode. A 25 mVolt overpotential was applied to the electrode at a frequency of 10 Hz.

### Example 1

This example describes an assay to screen for inhibitors of farnesyl protein transferase (FPT) and geranylgeranyl protein transferase (GGPT).

We have designed an assay that can be readily multiplexed that: 1) incorporates the wild-type target protein and mutants into the initial screening process; 2) directly detects and quantitates the addition of farnesyl/geranyl groups and analogs thereof to the target protein by the enzyme of interest; and 3) can detect differential effects of drug candidates on enzyme activity.

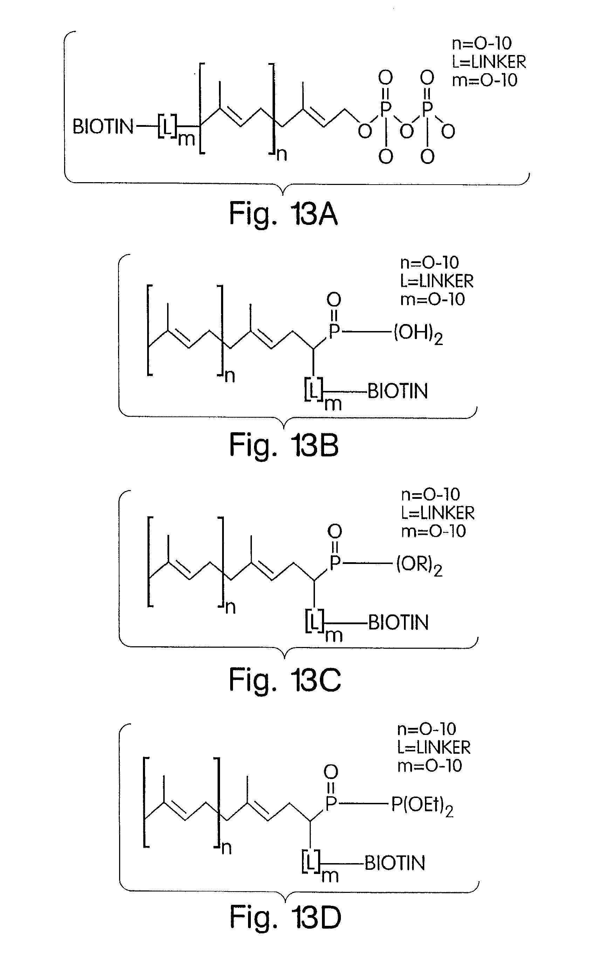

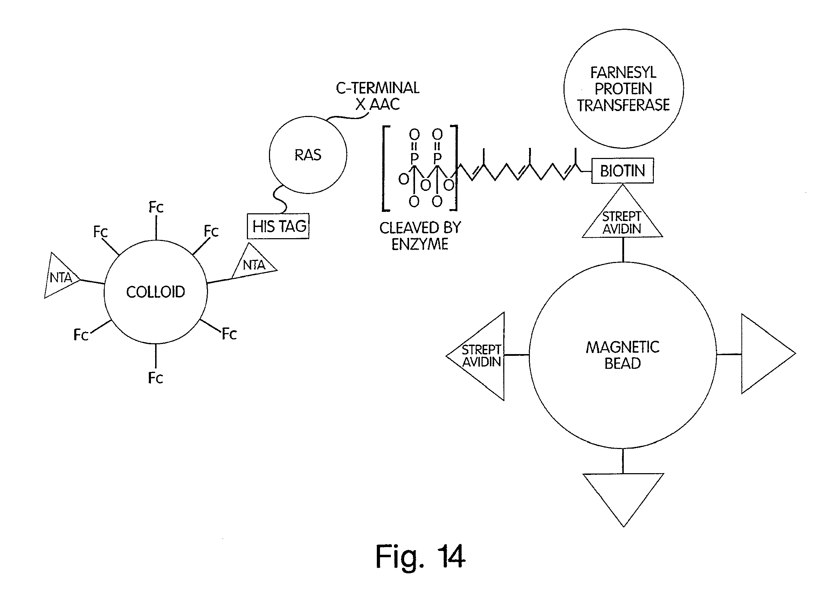

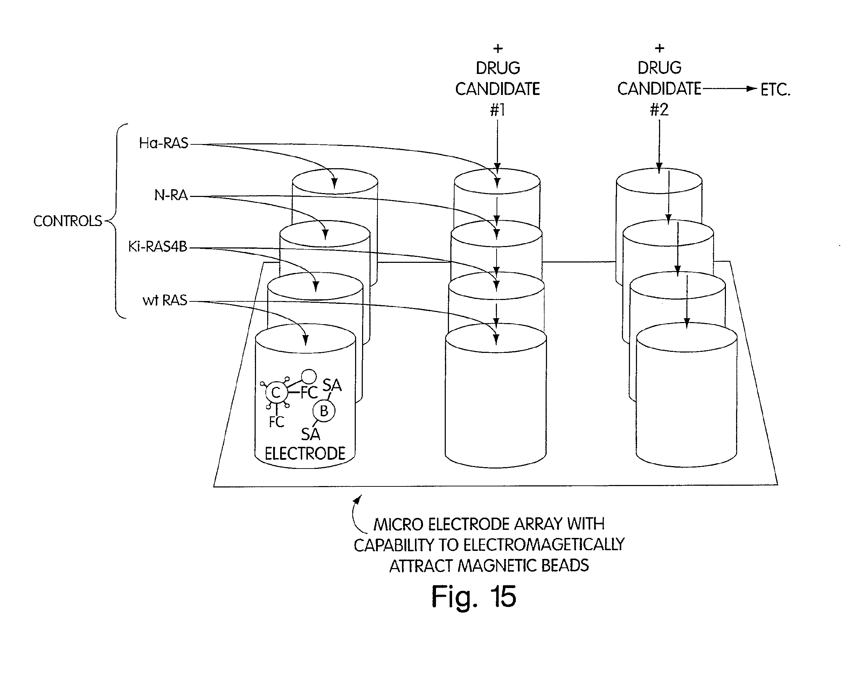

Our strategy is to: 1) attach histidine-tagged RAS proteins (or peptide motifs or fragments derived from the various mutants) to colloids bearing SAMs that present both NTA/Ni(II) groups (to capture histidine-tagged proteins) and transition metals such as ferrocene for electronic or electrochemical signaling; 2) modify farnesyl pyrophosphate derivatives with biotin (Figure 13); 3) add enzyme; 4) add candidate inhibitor; 5) add magnetic beads bearing streptavidin; 6) magnetically attract to a sensing electrode and electronically analyze. The enzyme FPT should add the biotinylated farnesyl (or geranyl) moieties to the RAS immobilized on the signaling colloid. Magnetic beads bearing streptavidin will bind to the biotin and the complex will be both recruitable and detectable. Assays will be performed in parallel in microwells interfaced with a microelectrode array, varying the RAS variant immobilized on the signaling colloid, and the drug candidate (Figures 14 and 15). The entire microelectrode array will be electronically analyzed to determine the differential effects of drug candidates on enzyme activity for particular target proteins. If a drug candidate interferes with the enzyme adding a farnesyl group to the target mutant protein, a diminution of the electronic or electrochemical signal will result.

This strategy can be used to monitor an enzyme's activity or screen for drugs that modulate its activity. Non-histidine-tagged proteins can also be attached to signaling colloids by standard coupling chemistry. Farnesyl derivatives can alternatively be modified with a recognition group other than biotin, that is a binding partner of a group immobilized on the magnetic beads.

Edelstein and Distefano have reported that farnesyl pyrophosphate groups modified with photoactivatable cross-linking moieties can be added to peptide motifs derived from RAS by yeast FPT (Edelstein R. and Distefano M. (1997). Photoaffinity labeling of yeast farnesyl protein transferase and enzymatic synthesis of a RAS protein incorporating a photoactive isoprenoid. Biochem. and Biophys. Res. Comm. 235, 377-382). Their objective was to use this assay to show that farnesyl derivatives were recognized by FPT, by cross-linking the two. This finding is consistent with the idea that farnesyl or garenyl moieties can be modified with biotin at the end distal from the pyrophosphates without interfering with enzyme activity or specificity.

### Example 2

In this example, cells were attached to gold-coated electrodes derivatized with SAMs. The cells, which were still attached to the electrode, were then incubated with a solution containing gold colloids which had been derivatized to present both a ligand specific for a receptor on the cell surface and a redox-active metal capable of delivering an electronic or electrochemical signal to the electrode. After some incubation period, the electrodes were scanned by alternating current voltammetry (ACV). A positive interaction between the colloid-bound ligand and the cell surface receptor will bring the redox-active metal, also on the colloid, close enough to the electrode to transduce an electronic or electrochemical signal.

More specifically, electrodes were derivatized with SAMs to present 10% methyl head groups in a background of 50% Bis(ethynylphenyl thiol) (i.e. C₁₆H₁₀S) to facilitate electron flow to the electrode. 40% triethylene glycol-terminated thiols (HS(CH)₂)₁₁(CH₂CH₂)₃OH) were included to help monolayer packing. It had previously been shown that cell growth can be supported on HSC15-methyl-terminated SAMs that were coated with collagen. Both the HSCH₂C₁₅CH₃ and the collagen are insulating molecules and can inhibit electron flow to the electrode. For this reason, in this example, the saturated carbon chain-collagen coverage was reduced to produce islands of growing cells adjacent to the more conducting molecular wires. Human endothelial cells (HUVECs), that present a cell surface receptor αVβ3, which is important for angiogenesis, were grown on the electrodes. SAM-coated gold colloids bearing a ligand for the receptors and ferrocene moieties for electronic or electrochemical signaling were briefly incubated with the cell-presenting electrodes, then analyzed by ACV.

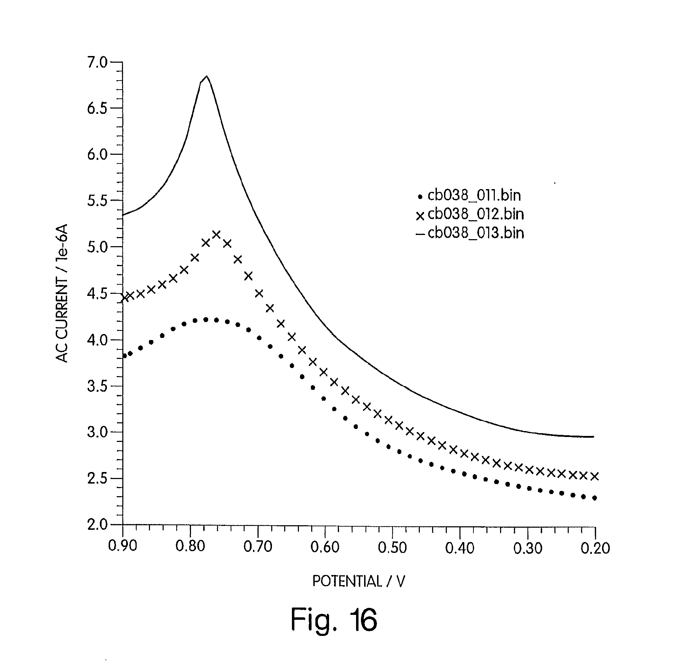

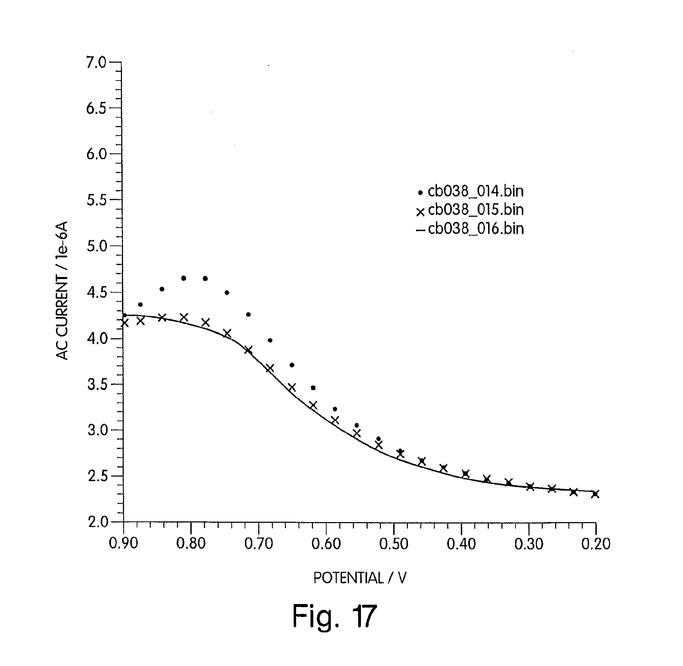

For ACV analysis, a 1ml capacity silicone gasket was clamped onto the cell-presenting electrode. 100ul of NTA-Ni colloids that had been pre-bound with a His-tagged RGD motif peptide and 100ul PBS were added to the gasket for incubation with the cell-presenting electrode. After 15 minutes, the first ACV scan was taken. Two successive scans were taken at 15 minute intervals. Current output was plotted against voltage. The first scan produced a broad current bulge characteristic of PBS buffer. The second and third scans generated a distinctive current peaks 0.5uAmps and 1.6uAmps respectively, at a characteristic ferrocene potential (at 780 mVolts), see Figure 16. The negative control, in which an identical electrode presenting the same cells was incubated with 100uL of NTA-Ni(II) colloids that were pre-bound with His-tagged GST, produced a first scan that was similar to the electrode incubated with RGD-presenting colloids. However, the broad bulge had decayed by the second and third scans which were identical (Figure 17). In sharp contrast to the 1.6 uAmp peak for the positive, no well-defined peak resulted for the negative control.

### Example 3: Cell growth on conducting surfaces

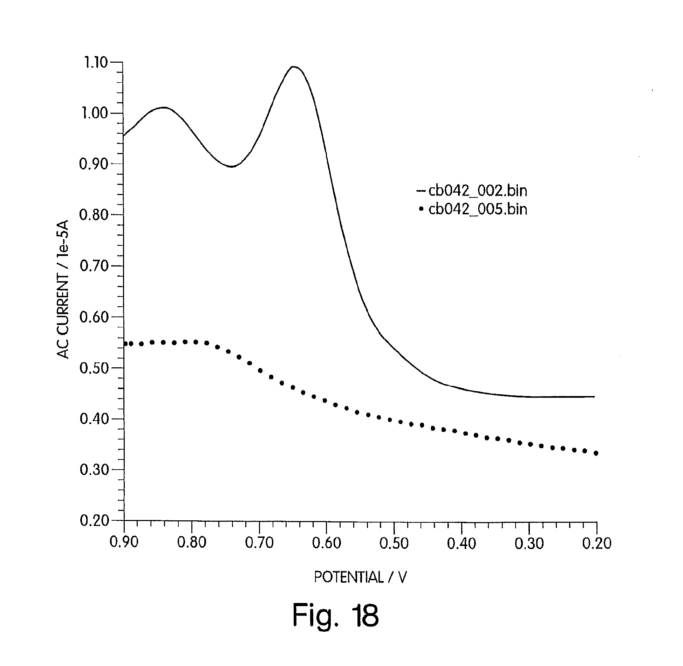

This example describes the electronic detection of cells grown on "conducting" surfaces that were not coated with collagen. Cells were grown on gold electrodes that were modified with sulfur-containing molecules, in some cases assembled into monolayers, but not coated with collagen. Electrode modification was performed as described in the electrode preparation section of Example 2, with the exception that electrodes incubated with 100% candidate molecule were not heat cycled in tri-ethylene glycol-terminated thiol. Several electrodes were assembled in the same cell growth flask and media containing HUVEC cells was added. The electrodes and cells were incubated in a C0₂ incubator for 24 hours. Surfaces were visually analyzed using 100X magnification. Table 1 lists surface modification and subsequent cell adhesion/growth characteristics. Surfaces showed low non-specific binding. Once cells bound, however, cell growth was good. Cells can easily be immobilized on a SAM by presenting a metal chelate coordinating a metal via the SAM, linking a protein to the SAM via a metal binding tag on the protein, the protein attracting the cell. Photographs were taken to document results (not shown). Cells were incubated with colloids (as described above) that displayed ferrocene moieties and a peptide, HHHHHH(S₄G₁)₃GRGDSGRGDS, that was specific for the αVβ3 receptor on the cell surface; or as a negative control, an irrelevant peptide, HHHHHH-Glutathione S-Transferase (GST). Figure 18 shows that cells grown on a 100% ethynylphenyl thiol (MF1) SAM-coated electrode produced current peaks only if incubated with colloids bearing the ligand specific for the αVβ3 receptor (Figure 18, solid line) and not when incubated with colloids derivatized with an irrelevant peptide GST (Figure 18, dotted line).

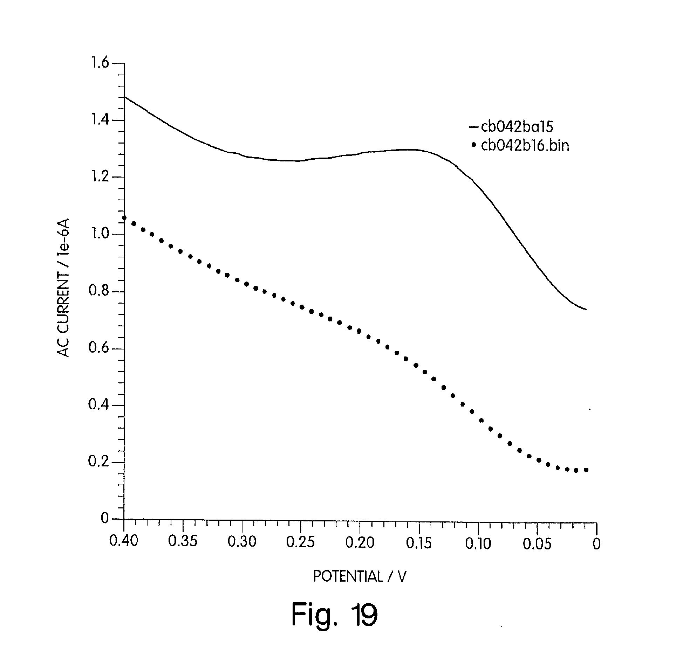

Figure 19 shows electrochemical scans of electrodes derivatized with 25% 2-mercaptoethylether in a background of insulating tri-ethylene glycol terminated thiols. Cells derivatized with colloids presenting the RGD sequence peptide produced a small peak (Figure 19, solid line), while the cells incubated with colloids presenting the GST peptide did not (Figure 19, dotted line).

**TABLE 1**

| **SURFACE MODIFICATION** | **CELL ADHESION within 1/2 hour** | **CELL GROWTH within 24 hours** |
|---|---|---|
| 100% methyl-terminated SAM | NO | NO |
| 100% methyl-terminated SAM, incubated with irrelevant proteins | NO | NO |
| 100% methyl-terminated SAM, incubated with collagen | NO | YES |
| 10% methyl terminated thiol in a background of molecular wire thiols, with or without tri-ethylene glycol-terminated thiols then coated with collagen | NO | YES |
| 100% tri-ethylene glycol-terminated SAM | NO | YES |
| 100% HS-2-unit molecular wire | NO | YES |
| 100% 2-mercaptobenzothiazole | NO | YES |
| 100% 1,2-benzenedimethanethiol | NO | YES |
| 100% Benzene ethane thiol | NO | YES |
| 100% 2-mercaptoethyl ether | NO | YES |
| NTA-Ni(II) in a background of tri-ethylene glycol-terminated thiol with or without ferrocene thiols mixed in- unbound by a His-tagged peptide | NO | YES |
| NTA-Ni(II) in a background oftri-ethylene glycol-terminated thiol with or without ferrocene thiols mixed in - bound by an irrelevant His-tagged peptide | NO | NO |
| NTA-Ni(II) in a background of tri-ethylene glycol-terminated thiol, with or without ferrocene thiols mixed in - bound by a cell specific His-tagged peptide | YES | YES |

### Example 4: Decoration of polymeric bead with colloid particles via Glutathione-S-Transferase / Glutathione binding

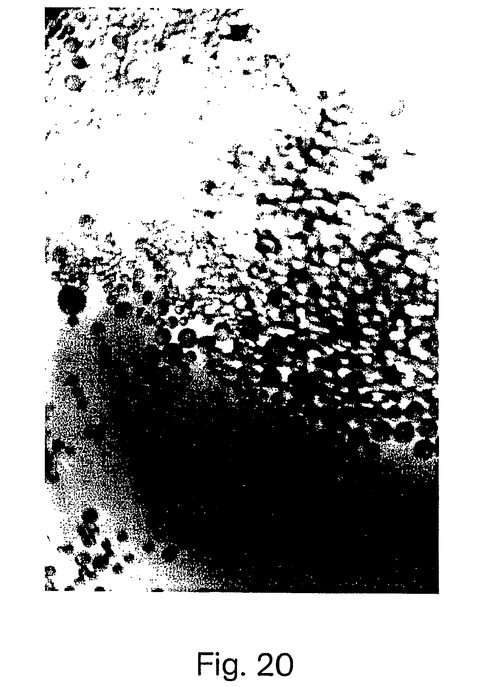

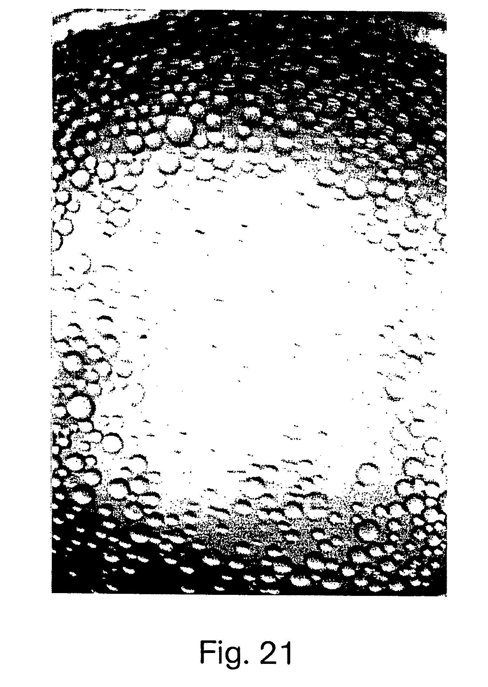

See Figures 20 and 21. The example below demonstrates how colloids presenting a target protein agglomerate onto a non-colloidal particle that presents a small molecule, drug candidate, peptide, protein, nucleic acid, or combinations thereof that is or are a binding partner of the target protein. Many combinatorial drug libraries are synthesized on non-colloidal particles or beads and can be mixed with colloidal particles that display a medically relevant target species. One can readily identify the bead that displays the drug that is a binding partner for the target species, as the colloidal particles agglomerate onto that bead and color it red.

A target protein, Glutathione-S-Transferase (GST) was histidine-tagged and immobilized on SAM-coated colloids that presented NTA-Ni (histamine tags bind NTA-Ni). 30ul of colloids presenting 40uM NTA-Ni on the surface were added to 65ul of 21.5uM GST, to give a final concentration of 14uM GST in solution. Glutathione, a small molecule that binds GST, is commercially available bound to agarose beads through Sigma-Aldrich. Glutathione-coated beads were incubated with the solution of GST-bound colloids. Within minutes, the GST bound to the glutathione beads, bringing the colored colloids out of solution, and decorating the beads red (Figure 20). Beads displaying a small molecule that does not bind to GST remained colorless when incubated with the GST-bound colloids (Figure 21). A second negative control, in which glutathione-coated beads were incubated with 30ul NTA-Ni colloids in the absence of GST showed that NTA-Ni-colloids do not bind nonspecifically to the bead surfaces or to the glutathione.

### Example 5: Demonstration of Control of SAM permeability to electrons

This example demonstrates the ability to form a SAM including enhanced electronic communication. The SAM is formed on a surface that includes a mixture of a first, tight-packing species and a second species of different molecular structure that enhances the permeability of the SAM to electronic communication. A defect, or opening, is formed in the SAM allowing fluid to which the surface is exposed to communicate electrically with the surface. Specifically, certain small sulfur containing compounds having disruptive structures relative to the SAM as a whole were stably incorporated into a SAM, and greater permeability to electrons was demonstrated. This example demonstrates that a surface can be made electrically relatively conductive, and then support cell growth.

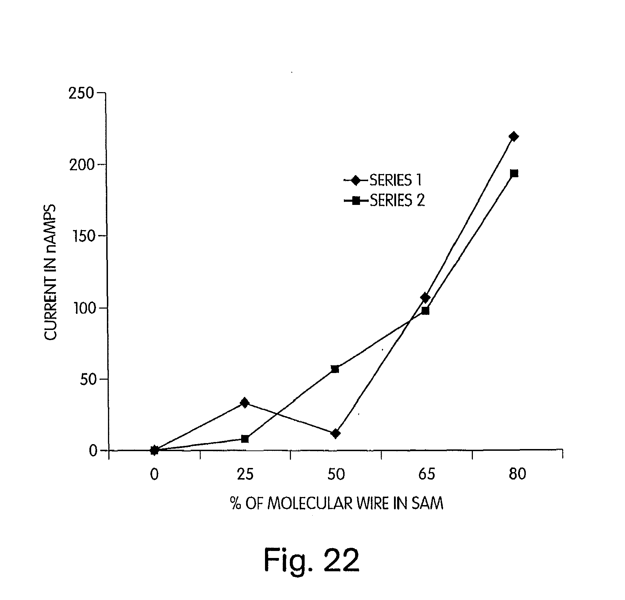

A water-soluble ferrocene derivative was dissolved in the electrolyte solution: 100mM solution of ferrocenedicarboxylic acid in 500uM NaClO₄. The working electrode was a gold-coated electrode derivatized with a SAM comprised of varying amounts of 2-unit molecular wire (MFI). The height of the peak at a characteristic ferrocene potential was plotted as a function of molecular wire density. As a negative control, a gold-coated electrode was derivatized with an insulating SAM comprised of 100% tri-ethylene glycol terminated thiol. Figure 22 shows that the "conductivity" of the SAM or the ability of electrons in solution to penetrate the SAM is a function of the density of 2-unit molecular wire integrated into the SAM. This system was used to test the conductivity of electrodes modified with a panel of sulfur-containing compounds. The compounds were dissolved in DMF at 50% candidate compound and 50% tri-ethylene glycol terminated thiol. Electrodes were derivatized as described in Example 1. Table 2 lists the candidate compounds and the height of the current peak produced when analyzed by ACV. Figure 22 shows two experimental results of tests of conductivity of a monolayer as a function of monolayer disruption by poly (ethynylphenyl thiol).

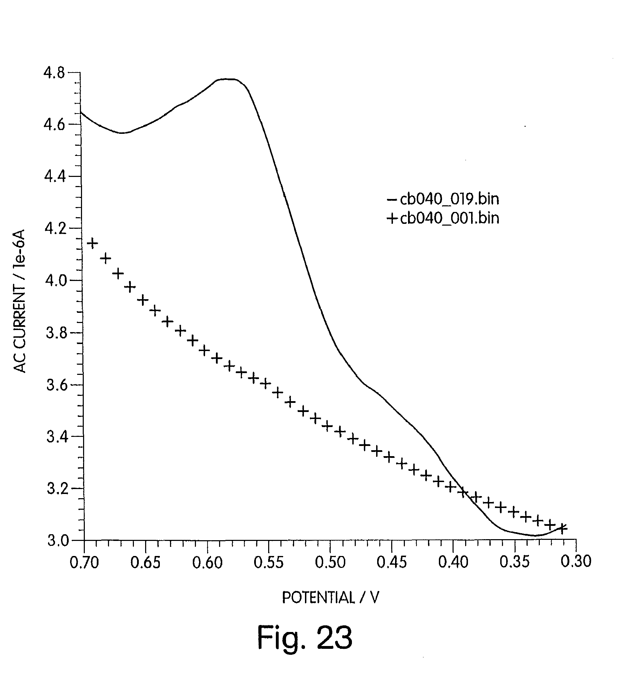

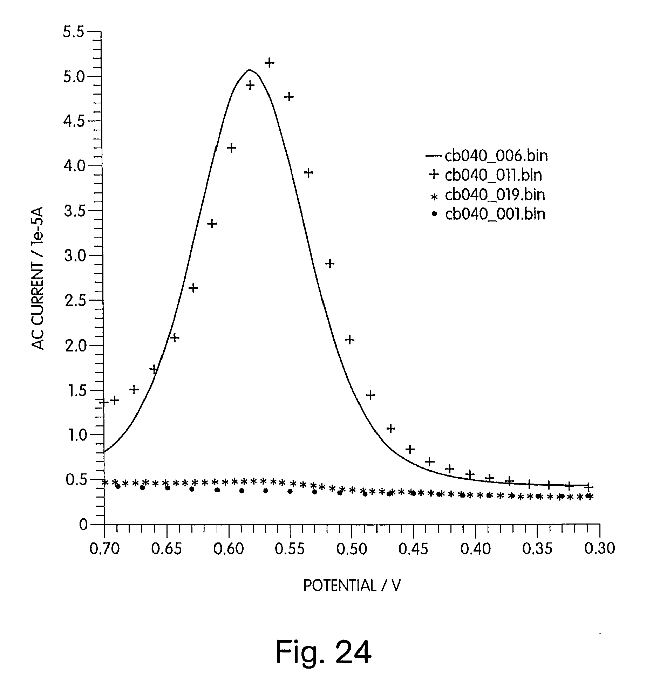

SAMs were formed on gold chips from 500 micromolar triethylene glycol-terminated thiol and 500 micromolar of either mercaptobenzothiazole or 2-mercaptoethyl ether in DMF. The chips were clamped between a flat substrate and a 1 ml capacity silicon gasket. A solution of ferrocene dicarboxyllic acid was dissolved in 500 micro molar NaClO4 and placed in the silicon gasket with a Ag/AgCl reference electrode and a Pt auxiliary electrode. The gold chip was connected as the working electrode. The system was analyzed by ACV. The magnitude of the current peaks, resulting from the ferrocene in solution communicating with the electrode, was an indicator of the ability of the trial compounds to make the SAM more permeable to electron flow by creating defects within the SAM. Figure 23 is an overlay of a SAM comprised solely of the insulating species tri-ethylene glycol-terminated (CH2)11-SH (+++ line) and the reportedly more conducting poly-ethynylphenyl thiol species (MF1) (solid line). The conducting poly-ethynylphenyl thiol species is expected to by more conductive, as an identical molecule but with two additional repeat units is reported to be conductive (Science 1997 Bumm et al). As predicted, the alkyl thiol SAM does not produce a current peak in a ferrocene dicaroxyllic acid solution, but a SAM comprised of 50% poly-ethynylphenyl thiol does. Figure 24 shows the plots of Figure 23 (dots are TEG-terminated thiol, stars are poly-ethynylphenyl thiol), against the more conducting SAMs including 2-mercaptoethyl ether (solid line) and mercaptobenzothiazole (+++ line), showing that the current peak from the poly-ethynylphenyl SAM is orders of magnitude smaller than those generated by SAMs comprised of 50% 2-mercaptoethyl ether or a SAM comprised of 50% mercaptobenzothiazole, which is consistent with the idea that SAMs can be made permeable to electron flow by either incorporating conductive species into the SAM or by inserting "defect" or "opener" molecules into the SAM.

**TABLE 2. Electrodes were derivatized with the following compounds in a background of tri-ethylene glycol-terminated thiol. Electrode preparation was performed as described in Example 1. The "conductivity" or permeability of the surfaces was assayed by measuring the magnitude of the current peak produced by the oxidation/reduction of ferrocene dicarboxyllic acid in the electrolyte solution (0.5 M NaClO4). The ability of each compound to resist non-specific binding was assayed by dipping each surface in BSA (bovine serum albumin) prior to measurement. Non-specifically adsorbed proteins would occlude the conduction of electrons through the monolayer to the electrode.**

| **COMPOUND** | **50% coverage Peak height in :Amps** | **25% coverage Peak height in :Amps** | **25% coverage Peak height in :Amps after blocking with BSA** | **Supports cell growth on 100% coverage or fractions thereof** |
|---|---|---|---|---|
| HS(CH**₂**)**₁₁** Tri-ethylene glycol | 0 | ND | ND | YES |
| **100% coverage -negative control** | | | | |
| HS-2-unit molecular wire | 0.415 | ND | 0.15 at 50% coverage | YES |
| **Positive control** | | | | |
| 2-mercaptopyridine | 14.70 | ND | ND | ND |
| 2-mercaptobenzothiazole | 44.00 | 0.28 | 0.29 | YES |
| Dithiothreitol | 41.00 | ND | ND | ND |
| 1,2-benzenedithiol | 40.00 | ND | ND | ND |
| 1,2-benzenedimethanet hiol | 34.90 | 28.00 | 4.02 | YES |
| Benzeneeth anethiol | 31.50 | 0.51 | 0.08 | YES |
| 2-mercaptoethyl ether | 39.30 | 57.70 | 19.0 | YES |

### Example 6: Detection of Protein-protein interactions

This example demonstrates the utility of a colloid particle having an immobilized signaling entity and an immobilized protein. (See Figure 27.)

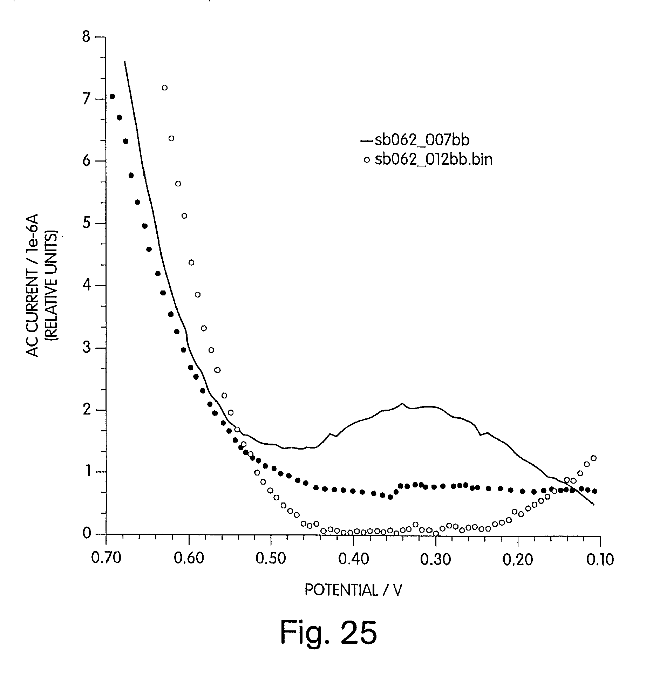

Histidine-tagged Glutathione-S-Transferase (GST-His) was attached to NTA-SAM-coated colloids, displaying 40uM NTA-Ni and 100uM ferrocene-thiol. Commercially available magnetic beads presenting protein A were coated at 1/10 binding capacity with anti-GST antibody, added at a 1:5 ratio to the GST-colloids, and measured on a 50% MF-1 SAM-coated electrode, which was placed on top of a magnet. The magnet pulled the magnetic beads onto the electrode surface to form a thick, visible precipitate. The GST-colloids were brought down to the electrode surface by the interaction with the GST-antibody on the magnetic beads to give a current peak at approximately 280mV. Two negative controls were run, one where GST was not attached to the colloid surface, and another where the GST antibody was not attached to the magnetic beads. Neither negative control gave a current peak. Figure 25 plots the results of this demonstration. Solid line represents interaction between GST-His-presenting colloids and anti-GST/Ab on magnetic beads. Open circles represent magnetic beads presenting the antibody incubated with colloids that did not present GST. Closed circles represent beads not presenting the antibody, incubated with colloids that presented GST.

### Example 7: Cell Detection

This example demonstrates both the advantage of forming a SAM on a surface that includes a mixture including a molecular species that enhances electronic communication across the SAM by forming a defect in the SAM allowing fluid to which the surface is exposed to communicate electrically with the surface, and the utility of attachment of a colloid carrying immobilized signaling entity to a protein. The protein is in turn immobilized at a cell attached to the surface of an electrode presenting the SAM. The defect in this case is caused by bulk of the a SAM-incorporated molecule including phenyl rings.

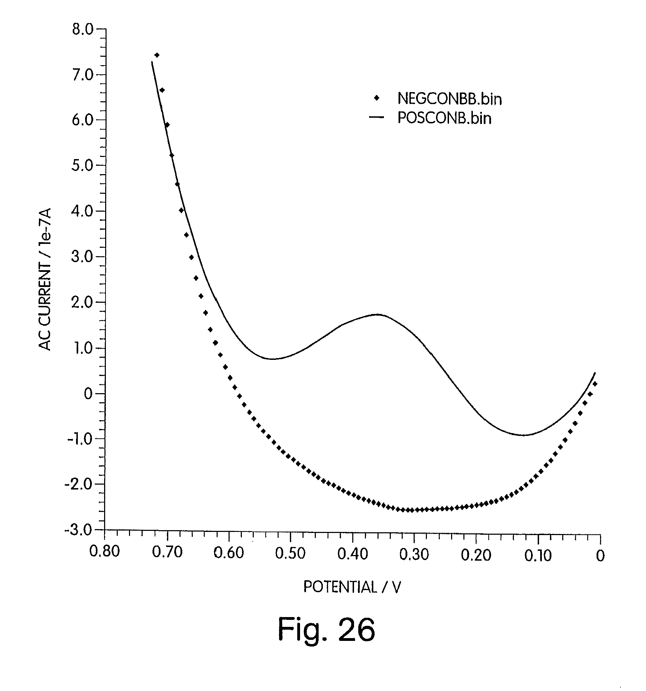

HUVEC cells were suspended in media and placed in a flask over a SAM coated on a gold surface. The SAM included 50% straight chain thiols, and 50% of the 2-unit poly (ethynylphenyl) thiol (MF1). 5ul of an 8.4mM RGD-His peptide solution was added to the media, and cells were incubated at 37C overnight to adhere to the electrode surfaces. After approximately 16 hours, 100ul of SAM-coated colloids, displaying NTA for capturing the RGD-His peptide and ferrocene for signaling, were added to the cells and incubated for 20 min at room temperature. The electrodes were then rinsed in buffer to wash off any unbound colloids and measured. Current peaks were recorded at 220-250mV. Negative controls were cells incubated with His-GST, an irrelevant protein that should not bind to cells. Colloids were added to negative controls, electrodes were rinsed in buffer, and measurements were taken. No peaks were observed for negative controls. Figure 26 shows a peak (solid line) generated when colloid presenting ferrocene signaling entity and his-tagged ligand to a cell surface receptor is brought to an electrode surface by cell/surface interaction. Diamond represent negative control where colloids displayed an irrelevant protein selected not to bind to the cell surface receptor.

### Example 8: Detecting Ligand-Receptor Interactions for Unmodified Ligands

This example demonstrates the ability to determine protein/ligand interaction in the absence of SPR without labeling either the protein or the ligand. Specifically, exposing a ligand to a protein suspected of interacting with the ligand where the ligand is in fixed proximal relationship with an electroactive entity, namely ferrocene, whose electroactive signal is dependant upon proximity to the protein, by forming a SAM including the ligand and ferrocene on the surface of an electrode.

We have found that the characteristic oxidation potential of a ferrocene derivative can be shifted based on the chemical nature of the ferrocene derivative's local environment. As proteins are brought into close proximity to the ferrocene derivative, a second current peak at an altered potential appears. The magnitude of this altered peak is proportional to the density of protein in the system.

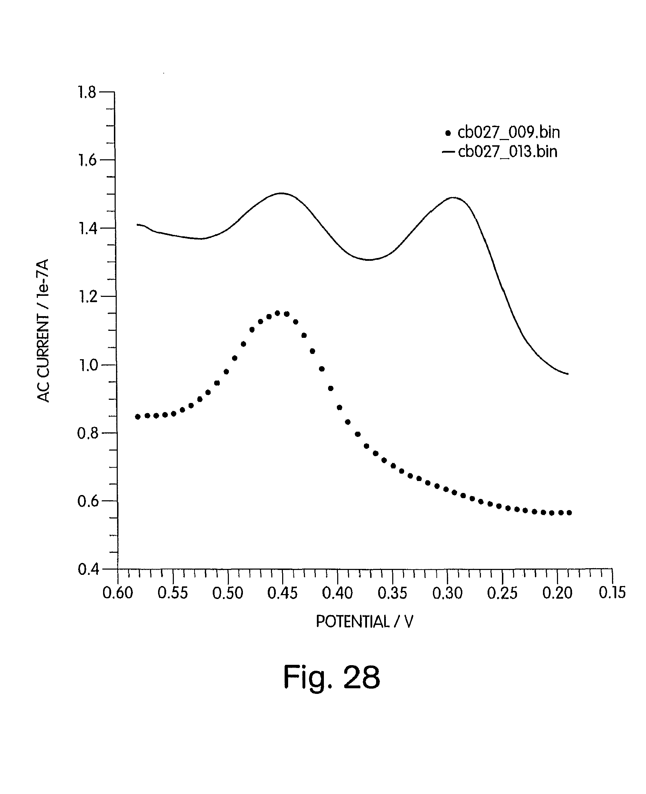

A panel of gold-coated electrodes were derivatized with heterologous SAMs that comprised a constant density of conductive molecular wire thiol and variable density of methyl-terminated thiol in a background of tri-ethylene glycol-terminated thiol. It had previously been shown that: 1) tri-ethylene glycol-terminated SAMs resist the non-specific adsorption of proteins; and 2) methyl-terminated SAMs bind proteins, specifically collagen. We therefore assumed that if the electrodes are incubated with collagen in solution, the density of collagen, that non-specifically adsorbs, will increase as the density of methyl-terminated thiol increased. SAMs that contained 2%, 4%, 10% and 15% methyl-terminated thiol were formed on gold-coated electrodes. Half the electrodes were incubated for 2 hours at 4°C with 0.005 mg/ml collagen in PBS while the other half was incubated with PBS alone. The electrodes were analyzed by alternating current voltammetry (ACV) with a water soluble ferrocene derivative, ferrocenedicarboxylic acid, dissolved in the electrolyte solution (0.5 M NaC104). The alternating oxidation/reduction of the ferrocene at a characteristic potential produces a current peak. The magnitude of this current peak and potential at which it occurred were recorded. At low density methyl-terminated thiol, a current peak was produced at about 450 mVolts; its magnitude and position appeared to be unaffected by the presence of the protein, collagen. At higher density methyl-terminated thiol, 10% and over, a second current peak is produced at about 300 mVolts, but only in the presence of the protein collagen. Figure 28 shows high-density methyl terminated thiol electrodes in the presence of ferrocene dicarboxylic acid. Solid line represents electrodes with collagen bound to the methyl thiols. The hydrophobic environment caused by the collagen on the surface of the electrode causes the oxidation potential of the ferrocene to shift, and two peaks are seen. Solid circles represents high-density methyl terminated thiol electrodes that are not bound to collagen and thus do not affect the oxidation of the ferrocene.

This strategy can be used to detect the presence of unmodified proteins, peptides, cells as follows: a mixed SAM is formed from ferrocene dicarboxyllic acid attached to an alkyl thiol, a thiol designed to make the SAM permeable to electron flow and a thiol terminated in a binding partner for a target species (the binding partner can also be histidine-tagged and attached to the electrode by binding to an NTA-Ni moiety in the SAM). If the target species is present in the sample solution, then it binds to its binding partner that is presented on the SAM. The presence of the protein near the SAM surface changes the chemical environment around the ferrocene dicarboxyllic acid and causes its oxidation potential to shift to a lower potential. A shift in the characteristic oxidation potential of the immobilized redox-active metal indicates the presence of the target species.

When these experiments were performed with ferrocene derivatives that did not have polar substituents on the ring, no effect was observed. Therefore, for this strategy to work, it is essential that the immobilized redox-active metal is in a polar environment and that the immobilized binding partner (connected to the SAM via a thiol) is as small as possible.

### Example 9: ELISA

A technique familiar to those skilled in molecular or cellular biology is Enzyme-linked Immunosorbent Assays (ELISA) *(*Current Protocols in Molecular Biology, Volume 2, Immunology 11.2, 1996, copyright from John Wiley and Sons Inc. 1994-1998). A major problem with ELISAs is sensitivity. Enzyme signaling occurs at a 1:1 ratio; one antibody-coupled enzyme signals the presence of one antigen. Therefore, microgram quantities of antigen are necessary for an effective ELISA, which is not plausible in all cases. This renders ELISAs ineffective for detection of antigens on a cell surface that are expressed or presented at low levels. Another problem with ELISAs is that at low antigen concentrations, the assay is very time-consuming. The amount of enzyme hydrolysis is directly proportional to the time of hydrolysis. Normally when one performs an ELISA, a target species is directly or indirectly attached to a plastic substrate. The presence of the immobilized species is then detected by binding to it a "secondary" antibody that also has a signaling capability; the secondary antibody is usually conjugated to an enzyme, typically horseradish peroxidase (HRPO), alkaline phosphatase (AP), or a fluorescent tag which is capable of performing a reaction on an added substrate that results in a color change (detected by a spectrophotometer), or a fluorescence labeling tag that can be detected by a fluorimeter (see, for e.g., "Localization of a passively transferred human recombinant monoclonal antibody to herpes simplex virus glycoprotein D to infected nerve fibers and sensory neurons in vivo", Sanna PP, Deerinck TJ, and Ellisman MH,1999, Journal of Virology Oct. Vol 73 (10 8817-23)). Most often, for convenience, so that every antibody does not need to be conjugated to an enzyme, a mouse antibody is used as the specific recognition antibody, then an enzyme-conjugated rabbit-anti-mouse antibody is added.

Using the technology described herein, one can greatly increase the sensitivity of ELISAs and detect the presence of the immobilized target species using a natural ligand (protein or peptide) or a drug candidate as the probe molecule. The presence of the immobilized species is detected by binding to it a *ligand* attached to a colloid that also presents a plurality of horseradish peroxidase (HRPO) or alkaline phosphatase (AP). The enzyme can be conveniently linked to the colloid by a variety of means, including a histidine-tag attached directly to the enzyme, or by binding a mouse-anti-goat enzyme-conjugated antibody to a goat antibody that is attached to the colloid via a histidine-tagged protein G (Akerstom, B., Nielson, E., Bjorck, L. Journal of Biological Chemistry, 1987 Oct. 5 Vol. 262 (28); pgs. 13388-91 and Fahnestock, S.R., Alexander, P., Nagle, J. and Filpula, D. (1986) Journal of Bacteriology Vol. 167, 870-880). By binding a ligand co-immobilized on a colloid with a plurality of enzymes, to the target species *in place of* a secondary antibody, the ratio of signaling molecules to binding events is increased by orders of magnitude. The binding of one antibody or ligand on the colloid to a presented antigen on an ELISA plate indirectly results in the binding of thousands or millions of enzymes. Alternatively, a known species can purposely be attached to wells of a 96-well plate so that one can probe with colloids that each present a separate drug candidate along with the signaling enzymes. Currently, it is not possible to do this with existing ELISA technology each drug candidate can not be conjugated to an enzyme. Alternatively, the natural ligand for the immobilized target can be presented on the colloid along with the signaling enzymes and drug candidates added to each well of the plate to disrupt the interaction. Unbound colloids and thus their signal are lost in a wash step. The target for the antibody- or ligand-presenting colloids can be a cell or a protein bound directly or indirectly (via another antibody or ligand) to an ELISA plate. The advantage to this modification of an ELISA is not only sensitivity, but also efficiency. Because several hundred signaling enzymes remain bound via the colloids to one antigen, substrate hydrolysis will occur more quickly, and less time will be needed for an adequate reading.

### Example 10: Visual Detection of colloid-immobilized ligand interaction with cell surface receptors and disruption thereof

This example was carried out in a manner as described in Example 3 with the following exceptions. Cells were grown on a multi-well plate. Following interaction, visual inspection showed decoration of the cells with colloids selectively at locations on the cell where the receptor was expressed. Referring to Fig. 29A, a control is shown in which no binding occurred. A random sequence peptide was used. Fig. 29B shows decoration of cells with colloids selectively at locations on the cells where protein was expressed.

### Example 11: Detection of binding interaction between binding partners, one bound non-specifically to a solid support, the other to a colloid

A protein, specifically streptavidin (Promega) was non-specifically attached to a solid support, specifically Q-sepharose beads. Its binding partner, biotin, was covalently attached to a thiol incorporated into a self-assembled monolayer on colloid particles. Exposure of the colloid particles to the beads resulted in immobilization via binding between the biotin and the streptavidin, coloring the beads red. Fig. 30A is a photocopy of a computer-digitized microscope image of beads decorated (red, originally) by the binding interaction. Fig. 30B (control), shows a similar image of beads that were not decorated: similar beads (streptavidin-bound) exposed to colloids presented an irrelevant protein.

This example also, importantly, describes a self-assembled monolayer comprising biotin on a colloid particle. Biotin SAMs were formed on colloids as follows. A solution in DMF was made including 580 micromolar COOH terminated thiol and 20 micromolar biotin-terminated thiol. Thiols were C11. Colloid preparation was carried out as described above, in which the described DMF solution of 20 micromolar biotin and 580 micromolar COOH thiols were incubated with the colloids. Other steps were as above.

### Example 12: ELISA with multiple signaling of a single binding_event

This example was run similarly to typical ELISA assays with the following exceptions/details: detergents and surfactants were omitted from wash steps. GST was immobilized onto a multi-well plate. Mouse anit-GST antibody was added to the wells and bound to GST. After a typical wash step, in one set of wells (experimental) colloids presenting histidine-tagged Protein G were added and bound to anti-GST. A second set of wells (control) was not exposed to the Protein G-immobilized colloids. Following another typical wash step, all wells were exposed to horse radish peroxidase-linked goat anti-mouse antibody. All were then exposed to typical horse radish peroxidase substrate and sufphuric acid. The reaction was allowed to develop in the normal manner. Plates were analyzed in a multi-well spectrophotometer reader.

The result of this example shows six orders of magnitude greater signaling in the experimental wells compared to the control wells, as horse radish peroxidase linked to multiple G locations on colloid particles. Fig. 31 is a bar graph showing this six order of magnitude increase. This example shows signaling of a single binding of a first biological or chemical agent to a second biological or chemical agent with a plurality of signaling entities. The signaling of the plurality of entities occurs simultaneously as they bind to the colloid particle.

### Example 13: Attachment of Unmodified Chemical or Biological Molecules to Self Assembled Monolayers Presented on Surfaces of Colloids, Specifically, Attachment of Streptavidin to Colloids

This example provides a technique for the attachment of essentially any amine-containing entity to a surface able to present a carboxylic acid or salt thereof. The technique can be used to attach the entity to a colloid, via attachment to a self-assembled monolayer formed on a surface of a colloid. The molecule that is attached to the self assembled monolayer need not include any particular chemical functionality, such as an affinity tag or the like, prior to attachment. The only requirement is that the molecule include at least one primary amine, such as are found in many amino acids. Accordingly, the technique is particularly well-suited for the covalent attachment of proteins or peptides that carry primary amino groups to colloids that present carboxylic acids at their surfaces.

Techniques for formation of self-assembled monolayers on colloids are described above. These techniques were followed, with the following specific details and provisions.

A protein was attached by forming an amide bond between a carboxylate on a colloid surface (attached via a SAM) and an amine residue of the peptide. The amide bond was formed by the application of a modified EDC/NHS coupling protocol. The success of the coupling was tested by mixing colloids presenting streptavidin with colloids presenting biotin groups (via a biotin SAM, described previously). If the streptavidin was successfully attached then the two types of colloids would aggregate and the suspension would change color and, ultimately, the colloids would precipitate out leaving a clear solution.

The colloids used for the coupling procedure were prepared as described elsewhere and were formed by incubation in a thiol solution containing 540uM (micromolar) COOH-terminated thiol, 60uM NTA-terminated thiol, and then heat cycling in 400uM ethylene glycol-terminated thiol. 50ul of colloids in PBS were treated with 50ul of 10mM EDC, 40mM NHS in water. After 7 min the colloids were spun down, the liquid was removed, and the colloids were resuspended in 500ul (microliter) of O.1mg/ml streptavidin in phosphate buffer, pH 6.4. After 1.5 h, unreacted sites on the colloids were blocked by adding ethanolamine and the colloids were spun down, then resuspended in 100 microL of pH 6.4 phosphate buffer to wash away excess, uncoupled streptavidin. Colloids were then spun down again and resuspended in 50ul PBS.

To a mixture of 20 microL of phosphate buffer and 15ul of biotin-presenting colloids was added 15ul of streptavidin-presenting colloids prepared above. This suspension was compared to a mixture of 35ul of phosphate buffer and 15ul of biotin-presenting colloids. The addition of streptavidin-presenting colloids caused an immediate change in color from red to blue. After ∼10 min the mixture of biotin colloids and streptavidin colloids appeared clear with precipitate on the bottom. The mixture that did not contain the biotin colloids remained red with no visible change.

The above-described protocol is applicable to attachment of essentially any chemical or biological molecule including an amine to a self-assembled monolayer on a colloid. To ensure binding of a molecule to only one colloid, rather than binding to multiple colloids which could result in aggregation, the following adjustments can be made if appropriate. The concentration of colloid in solution can be decreased, while maintaining concentration of molecule desirably attached and maintaining concentration of EDC/NHS reactant. Other slight modifications can be made by those of ordinary skill in the art for various molecules. Amine-containing molecules can be selected by those of ordinary skill in the art and include, without limitation, proteins, synthetic molecules, peptides, dervatized nuclic acids, and other dervatized or naturally-occurring biological molecules that contain amines. As would be appreciated by those of ordinary skill in the art, a wide variety of species can be synthezised with or attached to amines or amine-containing species to render them attachable to a colloid according to this embodiment of the invention.

Those skilled in the art would readily appreciate that all parameters listed herein are meant to be exemplary and that actual parameters will depend upon the specific application for which the methods and apparatus of the present invention are used. It is, therefore, to be understood that the foregoing embodiments are presented by way of example only and that, within the scope of the appended claims and equivalents thereto, the invention may be practiced otherwise than as specifically described.

Aspects (A) of the present invention are listed in the following pages.
1. A method comprising:
   allowing a colloid particle the ability to become immobilized with respect to a non-colloidal structure; and
   determining immobilization of the colloid particle relative to the non-colloidal structure.
2. A method as in A1, wherein the colloid particle comprises an auxiliary signaling entity.
3. A method as 2, wherein the auxiliary signaling entity comprises a dye, pigment, electroactive molecule, chemiluminescent moiety, electrochemiluminescent moiety, fluorescent moiety, up-regulating phosphor, or enzyme-linked signaling moiety including horse radish peroxidase and alkaline phosphatase.
4. A method as in 3, wherein the signaling entity comprises a metallocene.
5. A method as in 4, wherein the signaling entity comprises ferrocene or a ferrocene derivative.
6. A method as A1, comprising allowing a plurality of colloid particles to fasten to the non-colloidal structure, and determining fastening of the plurality of particles to the non-colloidal structure.
7. A method as in 6, wherein the plurality of colloid particles comprises auxiliary signaling entities.
8. A method as in 7, wherein the auxiliary signaling entities comprise a dye, pigment, electroactive molecule, chemiluminescent moiety, electrochemiluminescent moiety, fluorescent moiety, up-regulating phosphor, or enzyme-linked signaling moiety including horse radish peroxidase and alkaline phosphatase.
9. A method as in A1, further comprising providing a biological or chemical agent linked to or adapted for linkage to the non-colloidal structure, and a binding partner of the biological or chemical agent linked to or adapted for linkage to the particle, the allowing step comprising allowing the particle to become linked to the non-colloidal structure via the agent and the binding partner.
10. A method as in A1, wherein the non-colloidal structure is a bead.
11. A method as in 10, wherein the bead comprises polymeric material, agarose, tentagel, and/or magnetic material.
12. A method as in 11, wherein the bead is a polystyrene bead.
13. A method as in 9, comprising allowing the agent to be linked to the non-colloidal structure, the binding partner to be linked to the particle, and the agent and the binding partner to bind to each other.
14. A method as in 13, comprising allowing the agent and the binding partner to biologically bind to each other.
15. A method as in 9, wherein the biological or chemical agent is a drug candidate, and the binding partner is a target of the drug candidate.
16. A method as in 15, wherein the non-colloidal structure is a bead.
17. A method as in 15, wherein the non-colloidal structure is a surface of an essentially planar substrate or chip.
18. A method as in 9, wherein the biological or chemical agent is a nucleic acid sequence.
19. A method as in 9, wherein the biological or chemical agent is a peptide, and the binding partner is a binding partner of the peptide.
20. A method as in 9, wherein the biological or chemical agent is a protein, and the binding partner is a binding partner of the protein.
21. A method as in A1, wherein the colloid particle carries an immobilized ligand, and the non-colloidal structure carries a binding partner to the ligand, the method comprising allowing the colloidal particle the ability to fasten to the non-colloidal structure in the presence of a candidate drug for interruption of binding of the ligand to the target.
22. A method as in A1, wherein the non-colloidal structure is a bead, further comprising providing a plurality of beads, a plurality of biological or chemical agents adapted for linkage to the beads, a plurality of particles, and a plurality of binding partners of the biological or chemical agents adapted for linkage to the particles, wherein at least some of the agents and the binding partners are suspected of having the ability to bind to each other, the method comprising exposing at least some of the beads to at least some of the particles, and determining immobilization of the particles on the beads.
23. A method as in 22, wherein the biological or chemical agents are drug candidates and the binding partners are targets of the drug candidates, the method comprising providing at least a first and a second bead in two separate locations, each carrying a different immobilized drug candidate, exposing the plurality of particles to the at least two beads, and differentiating linkage of the particles to the first bead versus the second bead.
24. A method as in 23, wherein the at least two beads are separately located in at least two different wells of a multi-well plate.
25. A method as in 22, wherein the biological or chemical agents are drug candidates and the binding partners are targets of the drug candidates, the method comprising providing at least two beads carrying a drug candidate in two separate locations, exposing the first bead to a first set of colloid particles carrying a first target of the drug candidate, and exposing the second bead to a second set of colloid particles carrying a second target of the drug candidate, and differentiating linkage of the first set of particles to the first bead versus the second set of particles to the second bead.
26. A method as in 9, comprising determining immobilization of the particle on the non-colloidal structure by determining a change in spectrum of absorbed or transmitted electromagnetic radiation interacting with the particle.
27. A method as in 9, comprising determining immobilization of the particles on the non-colloidal structure by visual inspection.
28. A method as in 22, comprising providing a plurality of the beads and agents linked to the beads, a plurality of the particles and binding partners linked to the particles, and exposing the particles to the beads and determining immobilization of the particles on the beads.
29. A method as in 9, wherein at least one of the agent or binding partner is adapted for linkage to the non-colloidal structure or particle, respectively, via an affinity tag/binding partner linkage.
30. A method as in 9, wherein at least one of the agent or binding partner is adapted for linkage to the non-colloidal structure or particle, respectively, via a metal binding tag/metal/chelate linkage.
31. A method as in 30, wherein at least one of the agent or binding partner carries immobilized thereto a chelate coordinating a metal, and at least one of the agent or binding partner is derivatized with a polyamino acid tag.
32. A method as in 9, wherein at least one of the agent or binding partner is adapted for linkage to the non-colloidal structure or particle, respectively, via a self-assembled monolayer.
33. A method as in 9, wherein at least one of the agent or binding partner is adapted for linkage to the bead or particle, respectively, via complementary nucleic acid sequence pairs.
34. A method as in 9, wherein the binding partner is adapted for linkage to the particle via glutathione/glutathione-s-transferase ligand interaction.
35. A method as in 9, comprising:
   providing at least a first and a second non-colloidal structure comprising polymeric beads and at least a first and a second agent linked to the first and second beads, respectively;
   providing a plurality of colloid particles each carrying immobilized thereto a suspected binding partner of the first and/or second agent;
   exposing the beads to the particles; and
   differentiating linkage of the particles to the first bead vs. the second bead.
36. A method as in 35, wherein the first and second agents linked to the first and second polymeric beads are suspected of biological or chemical interaction with the binding partner, and the differentiating step comprises differentiating biological interaction between the first agent and the binding partner vs. the second agent and the binding partner.
37. A method as in 9, comprising:
   providing a plurality of non-colloidal structures comprising beads each carrying the agent immobilized thereto;
   providing a first set and a second set of colloid particles, the first set each carrying immobilized thereto a first suspected binding partner of the agent and the second set each carrying immobilized thereto a second suspected binding partner of the agent;
   exposing at least a first of the beads to the first set of particles and at least a second of the beads to the second set of particles;
   differentiating linkage of the first set of particles to the first bead vs. the second set of particles to the bead.
38. A method as in 37, wherein the first and second suspected binding partners are suspected of biological or chemical interaction with the agent, and the differentiating step comprises differentiating biological interaction between the agent and the first suspected binding partner vs. the agent and the second suspected binding partner.
39. A method as in A1, wherein the non-colloidal structure is a cell.
40. A method as in 39, further comprising providing a ligand for a receptor or protein at a surface of the cell, adapted for linkage to the particle, the allowing step comprising allowing the particle to be linked to the cell via the ligand interacting with the receptor or protein.
41. A method as in 39, wherein the ligand is adapted for linkage to the particle via a self assembled monolayer.
42. A method as in 40, wherein the ligand is a peptide, protein, antibody, enzyme, or small molecule.
43. A method as in 40, wherein the ligand is adapted for linkage to the particle via an affinity tag/binding partner linkage.
44. A method as in 40, wherein the ligand is adapted for linkage to the particle via a metal binding tag/metal/chelate linkage.
45. A method as in 42, wherein the ligand carries a polyamino acid tag and the particle carries an immobilized chelate coordinating a metal.
46. A method as in 45, wherein the chelate is nitrilotriacetic acid.
47. A method as in 45, wherein the particle carries a self-assembled monolayer including the nitrilotriacetic acid.
48. A method as in 45, wherein the particle carries a self-assembled monolayer including the immobilized chelate .
49. A method as in 40, comprising exposing the ligand and the particle to the cell, allowing the ligand to link to the particle, and determining fastening of the ligand to the receptor or protein.
50. A method as in 39, comprising determining immobilization of the particle on the cell by determining a change in spectrum of absorbed or transmitted electromagnetic radiation interacting with the particle.
51. A method as in 39, comprising determining immobilization of the particle on the cell electronically.
52. A method as in 51, comprising determining immobilization of the particle on the cell via alternating current voltammetry.
53. A method as in 50, comprising determining immobilization of the particle on the cell by visual inspection.
54. A method as in 39, comprising:
   providing a cell presenting a receptor or protein at a surface thereof;
   providing a colloid particle; and
   allowing the colloid particle to fasten to the receptor or protein.
55. A method as in 54, wherein the colloid particle includes an auxiliary signaling entity.
56. A method as in A1, wherein the non-colloidal structure is a cell exposing a receptor or protein at a surface thereof, further comprising providing a ligand for the receptor or protein adapted for linkage to the particle and exposing the ligand and the particle to the cell in the presence of a candidate drug for disruption of interaction between the ligand and the receptor or protein; and
   determining fastening of the particle to the cell.
57. A method as in 56, wherein the determining step involves determining inhibition of fastening of the particle to the cell indicative of effectiveness of the candidate drug in disrupting receptor or protein/target protein interaction.
58. A method as in 56, comprising providing at least a first and a second cell in separate locations, exposing the first cell to a ligand for a cell receptor or protein of the first cell and a colloid particle, the ligand adapted for linkage to the particle, and a first candidate drug for disruption of interaction between the ligand and the receptor or protein, and adding to the second cell the ligand and the colloid and a second candidate drug for disruption of interaction between the ligand and the receptor or protein, and differentiating linkage of the particle to the first cell versus the second cell.
59. A method as in A1, wherein the non-colloidal structure is a cell exposing a receptor or protein at a surface thereof, further comprising:
   providing at least two of the cells in separate locations;
   exposing each of the cells to a different target protein to a cell receptor or protein and a colloid particle adapted for linkage to the respective target protein; and
   determining fastening of the particles to the cells indicative of binding of the different target proteins to the cell receptor or proteins.
60. A method as in 9, wherein the non-colloidal structure is a magnetic bead and the colloid particle comprises an auxiliary signaling entity.
61. A method as in 60, wherein the signaling entity is a metallocene.
62. A method as in 60, wherein the signaling entity is ferrocene or a ferrocene derivative.
63. A method as in 60, comprising exposing the particle to the bead in the presence of the agent and the binding partner further in the presence of an enzyme having the ability to cleave the agent or binding partner.
64. A method as in 63, comprising first exposing the agent and the binding partner to the enzyme, then exposing the particle and the bead to the agent and the binding partner.
65. A method as in 63, comprising exposing the particle to the bead in the presence of the agent, the binding partner, and the enzyme further in the presence of a candidate drug for moderation of activity of the enzyme.
66. A method as in 65, wherein the binding partner comprises a protein or peptide that can be cleaved by the enzyme.
67. A method as in 66, wherein the protein is adapted for linkage to the colloid and to the bead.
68. A method as in 67, wherein the protein includes a metal binding tag and biotin, one of the colloid or the bead includes a chelate coordinating a metal, and the other of the colloid or bead includes streptavadin.
69. A method as in 67, wherein the protein includes a metal binding tag and biotin, the colloid includes a chelate coordinating a metal, and the bead includes streptavadin.
70. A method as in 60, wherein the signaling entity is a metallocene and the binding partner comprises a protein that can be cleaved by an enzyme, comprising exposing the particle to the bead in the presence of the agent, the binding partner, and the enzyme further in the presence of a candidate drug for moderation of activity of the enzyme, magnetically drawing the bead into proximity with an electrode, and determining proximity of the metallocene to the electrode by activating the electrode thereby determining effectiveness of the drug candidate in inhibiting cleavage activity of the enzyme.
71. A method as in A1, comprising exposing the colloid particle and the non-colloidal structure to an entity adapted for linkage both to the colloid particle and to the non-colloidal structure in the presence both of an enzyme having the ability to cleave the entity and a candidate drug for moderation of activity of the enzyme.
72. A method as in 71, wherein the non-colloidal structure is a magnetic bead and the colloid particle carries an immobilized electroactive species, the method comprising magnetically drawing the bead into proximity with an electrode, and determining proximity of the electroactive species to the electrode by activating the electrode thereby determining effectiveness of the drug candidate in inhibiting cleavage activity of the enzyme.
73. A method as in 71, wherein the non-colloidal structure is a surface of an electrode and the colloid particle carries an immobilized electroactive species, the method comprising exposing the electrode to the colloid particle, the entity adapted for linkage both to the colloid particle and to the electrode, the enzyme, and the candidate drug and determining proximity of the electroactive species to the electrode by activating the electrode thereby determining effectiveness of the drug candidate in inhibiting cleavage activity of the enzyme.
74. A method as in A1, comprising exposing the colloid particle and the non-colloidal structure to a substrate for an enzyme adapted for linkage to the non-colloidal structure, a molecular species linkable to the substrate via enzyme activity adapted for linkage to the particle, and an enzyme for the substrate.
75. A method as in 74, further comprising exposing the colloid particle and the non-colloidal structure to a candidate drug for moderation of activity of the enzyme.
76. A method as in 74, wherein the non-colloidal structure is a magnetic bead and the colloid particle carries an immobilized electroactive entity, the method comprising magnetically drawing the bead into proximity with an electrode, and determining proximity of the electroactive entity to the electrode by activating the electrode thereby determining effectiveness of the drug candidate in moderating activity of the enzyme.
77. A method as in 74, wherein the non-colloidal structure is a surface of an electrode and the colloid particle carries an immobilized electroactive entity, the method comprising exposing the electrode surface to the colloid particle, the substrate, the enzyme, and the candidate drug and determining proximity of the electroactive entity to the electrode by activating the electrode thereby determining effectiveness of the drug candidate in moderating activity of the enzyme.
78. A method as in 74, wherein the non-colloidal structure is a magnetic bead.
79. A method as in 78, wherein the colloid particle comprises an auxiliary signaling entity.
80. A method as in 78, wherein the signaling entity is a metallocene.
81. A method as in 80, wherein the signaling entity is ferrocene or a ferrocene derivative.
82. A method as in 78, comprising exposing the particle to the bead in the presence of the substrate and the binding partner further in the presence of a candidate drug for moderation of activity of the enzyme.
83. A method as in 82, wherein the binding partner is adapted for linkage to the particle via a metal binding tag/metal/chelate linkage and the substrate is adapted for linkage to the bead via biotin/streptavadin linkage.
84. A method as in 82, wherein the signaling entity is a metallocene, further comprising magnetically drawing the bead into proximity with an electrode, and determining proximity of the metallocene to the electrode by activating the electrode thereby determining effectiveness of the drug candidate in moderation of activity of the enzyme.
85. A method comprising:
   signaling a single binding of a first biological or chemical agent to a second biological or chemical agent with a plurality of signaling entities.
86. A method as in A85, comprising signaling the single binding with the plurality of signaling entities simultaneously.
87. A method as in A85, comprising providing the first agent carrying the plurality of signaling entities, allowing the first agent to bind to the second agent, and determining the binding via the signaling entities.
88. A method as in 87, wherein the first agent is linked to a particle to which the signaling entities are immobilized.
89. A method as in 87, wherein the first agent is linked to the signaling entities via a polymer.
90. A method as in 87, wherein the first agent is linked to the signaling entities via a dendrimer.
91. A method as in 87, wherein the first agent is a biological or chemical ligand, and the second agent is a cell presenting a receptor or protein at a surface thereof the method comprising allowing the ligand to fasten to the receptor or protein.
92. A method as in 87, wherein the plurality of signaling entities comprises a plurality of signaling entities linked to a polymer that is linked to the first agent.
93. A method as in 87, wherein the plurality of signaling entities comprises a plurality of signaling entities linked to a dendrimer that is linked to the first agent.
94. A method as in 87, wherein the first agent is fastened to a colloid particle that includes a plurality of immobilized signaling entities.
95. A method as in A85, wherein the plurality of signaling entities comprises a plurality of electroactive molecules.
96. A method as in 95, wherein the plurality of electroactive molecules comprises a plurality of metallocenes.
97. A method as in 95, wherein the plurality of electroactive molecules comprises a plurality of ferrocene or a ferrocene derivatives.
98. A method as in 92, wherein the polymer is linked to the first agent via an affinity tag/binding partner linkage.
99. A method as in 92, wherein the polymer is linked to the first agent via a binding tag/metal/chelate linkage.
100. A method as in 99, wherein the first agent carries a polyamino acid tag and the polymer carries a chelate coordinating a metal.
101. A method as in 93, wherein the dendrimer is linked to the first agent via a binding tag/metal/chelate linkage.
102. A method as in 101, wherein the first agent carries a polyamino acid tag and the dendrimer carries a chelate coordinating a metal.
103. A method as in A85, wherein at least one of the signaling entities comprises a dye, pigment, electroactive molecule, fluorescent moiety, up-regulating phosphor, or enzyme-linked signaling moiety including horse radish peroxidase and alkaline phosphatase.
104. A method comprising:
   determining protein/ligand interaction in the absence of SPR without labeling either the protein or the ligand.
105. A method as in A104, comprising exposing a ligand to a protein suspected of interacting with the ligand, the ligand in fixed proximal relationship with an electroactive entity having an electroactive signal dependant upon proximity to the protein.
106. A method as in A104, the electroactive entity having an electroactive signal that is alterable dependent upon altered proximity between the electroactive entity and the protein.
107. A method as in A104, comprising exposing a protein to a surface at which both the ligand and the electroactive entity are immobilized.
108. A method as in 107, wherein the surface is a surface of an electrode.
109. A method as in 107, wherein the ligand and electroactive entity each form part of a self-assembled monolayer at the surface.
110. A method as in 109, the self-assembled monolayer including a species that enhances permeability of the self-assembled monolayer to electrons.
111. A method as in 110, wherein the species that enhances permeability to electrons comprises a conductive self-assembled monolayer-forming species.
112. A method as in 110, wherein the species that enhances permeability to electrons comprises a species that causes defect sites in the self-assembled monolayer
113. A method as in A104, wherein the electroactive entity comprises a metallocene.
114. A method as in A104, wherein the electroactive entity comprises a ferrocene or a ferrocene derivative.
115. A method as in A104, wherein the electroactive entity comprises ferrocene dicarboxylic acid.
116. A method as in 109, wherein the ligand is linked to a self-assembled monolayer-forming species via an affinity tag/binding partner linkage.
117. A method as in 109, wherein the ligand is linked to a self-assembled monolayer-forming species via a metal binding tag/metal/chelate linkage.
118. A method, comprising:
   a) providing i) a solution comprising colloids, said colloids comprising a ligand capable of interacting with a cell surface molecule and ii) a composition comprising an electrode comprising growing cells, said cells comprising at least one cell surface molecule capable of interacting with said ligand,
   b) adding at least a portion of said colloids to said composition.
119. The method of A118, further comprising:
   c) detecting the aggregation of said colloids as a measure of the interaction of said ligand with said cell surface molecule.
120. The method of A118, wherein said colloid is a gold colloid.
121. The method of 120, wherein said gold colloid, prior to step (a), is treated so as to incorporate thiol groups.
122. The method of A118, wherein, prior to step (a), said ligand is derivatized with a moiety that has a binding partner.
123. The method of 122, wherein, prior to step (a), said ligand is derivatized with a moiety that can bind to a metal chelate.
124. The method of 123, wherein said moiety comprises a histidine tag.
125. A method, comprising:
   a) providing i) a solution comprising colloids, said colloids comprising a ligand capable of interacting with a cell surface molecule, ii) a candidate drug, and iii) a composition comprising an electrode comprising growing cells, said cells comprising at least one cell surface molecule capable of interacting with said ligand,
   b) mixing at least a portion of said colloids with said drug and said composition.
126. A method comprising:
   recruiting an electronic signaling entity to an electrode using a magnetic material.
127. A method as in A126, comprising recruiting the signaling entity to the electrode in part via protein/protein linkage involved in immobilization between the signaling entity and the magnetic material.
128. A method as 127, wherein the protein/protein linkage involves proteins that are not antibodies.
129. An article defining a surface, and a ligand suspected of interacting with a protein and an electroactive entity each immobilized relative to the surface.
130. An article as in A129, further comprising a species that enhances permeability of the surface to electrons immobilized relative to the surface.
131. An article comprising:
   a first biological or chemical agent, capable of biological or chemical binding to a second agent, immobilized relative to a plurality of signaling entities.
132. An article as in A131, wherein the first agent is linked to a particle to which the signaling entities are immobilized.
133. An article as in A131, wherein the first agent is immobilized relative to the signaling entities via a polymer.
134. An article as in A131, wherein the first agent is immobilized relative to the signaling entities via a dendrimer.
135. An article defining a surface, and a self-assembled monolayer formed on the surface of the article, the monolayer comprising a mixture of a first molecular species having a molecular structure promoting self-assembly at the surface with other first species in a tightly-packed manner preventing fluid to which the surface is exposed from communicating electrically with the surface, and a second molecular species having a molecular structure different from the first species in such a way to cause disruption of the tightly-packed self-assembled structure thereby defining defects in the tightly-packed structure allowing fluid to which the surface is exposed to communicate electrically with the surface.
136. An article as in A135, wherein the first species is essentially linear and the second species includes at least one non-linear portion.
137. A composition, comprising a first molecule and one or more signaling entities attached to a solid support, wherein said first molecule is a ligand capable of interacting with a cell-surface receptor or protein.
138. The composition of A137, wherein said solid support is a colloid.
139. The composition of 138, wherein said colloid is a gold colloid.
140. The composition of 138, wherein said ligand is covalently attached directly to said colloid.
141. The composition of 138, wherein said signaling entities are electroactive molecules.
142. The composition of 141, wherein said electroactive molecules comprise ferrocene or a ferrocene derivative.
143. The composition of A137, wherein said ligand is a peptide.
144. The composition of 143, wherein said peptide is derivatized with a moiety that can bind to a metal chelate.
145. The composition of 144, wherein said moiety comprises a histidine tag.
146. The composition of 144, wherein said solid support comprises a metal chelate and said peptide is attached to said solid support via binding of said moiety to said metal chelate.
147. The composition of A137, wherein solid support comprises a monolayer of a second molecule.
148. The composition of 147, wherein said monolayer is a self-assembling monolayer.
149. The composition of 147, wherein said second molecule is a thiol.
150. A composition, comprising a first molecule, a second molecule and a third molecule attached to a solid support, wherein said first molecule comprises a ligand capable of interacting with a cell-surface receptor or protein, wherein said second molecule forms a monolayer on said solid support, and wherein said third molecule is electroactive.
151. The composition of A150, wherein said solid support is a colloid.
152. The composition of 151, wherein said colloid is a gold colloid.
153. The composition of 151, wherein said ligand is covalently attached directly to said colloid.
154. The composition of 153, wherein said electroactive molecule comprises ferrocene or a ferrocene derivative.
155. The composition of 153, wherein said ligand is a peptide.
156. The composition of 155, wherein said peptide is derivatized with a moiety that can bind to a metal chelate.
157. The composition of 156, wherein said moiety comprises a histidine tag.
158. The composition of 156, wherein said solid support comprises a metal chelate and said peptide is attached to said solid support via binding of said moiety to said metal chelate.
159. The composition of A137, wherein said solid support is a liposome.
160. The composition of 147, wherein said liposome comprises at least one lipid containing a reactive group.
161. An article comprising a metal support constructed and arranged to support the growth of cells on a surface thereof, said metal support comprising a monolayer of at least one type of molecule, said monolayer configured such that metal support can be used as an electrode.
162. An article as in A161, further comprising cells growing on the metal support.
163. A composition comprising:
   a colloid particle
   a signaling entity immobilized relative to the colloid particle; and
   a protein immobilized relative to the colloid particle.
164. A species comprising:
   a polymer or dendrimer carrying a plurality of signaling entities adapted for linkage to a biological or chemical agent.
165. A species as in A164, wherein the polymer or dendrimer is adapted for linkage to the chemical or biological agent via a metal binding tag/metal/chelate linkage.
166. A species as in A164, wherein the polymer or dendrimer carries a chelate that can coordinate a metal.
167. A species as in A164, wherein the polymer or dendrimer carries a plurality of electroactive species.
168. A species as in A164, wherein the polymer or dendrimer carries a plurality of metallocenes.
169. A species as in A164, wherein the polymer or dendrimer carries a plurality of ferrocene or a ferrocene derivatives.
170. An article comprising a colloid particle immobilized relative to a glutathione derivative and at least one signaling entity.
171. An article comprising a colloid particle carrying on a surface thereof a self-assembled monolayer comprising a glutathione derivative.
172. A method as in A1, wherein the non-colloidal structure is a biological specimen.
173. A method as in 172, wherein the biological specimen is taken from a human or animal.
174. A method as in 173, wherein the biological specimen comprises cells or a tissue section.
175. A method as in 172, comprising providing at least a first and a second biological specimen;
   exposing the first and second specimens to colloid particles carrying immobilized species suspected of or having the ability to bind to binding partners presented by the specimens.
176. A method as in 175, wherein the first and second biological specimen of are different states of infection or disease.
177. A method as in 175, comprising determining a difference in binding of the colloid particles to the first specimen as opposed to the different specimen.
178. A method as in 177, wherein the difference comprises a difference in level of binding.
179. A method as in 177, wherein the difference is a difference in pattern of colloid immobilization.
180. A method as in 175, further comprising:
   determining immobilization of the colloid particles to either or both of the specimens
181. A method as in 175, wherein the biological specimen or its source has been pretreated with a candidate drug for modification of a disease state that can be determined by expression level and/or pattern of binding partners expressed by the specimen.
182. A method as in A1, wherein the non-colloidal structure comprises an article defining a surface, and a self-assembled monolayer formed on the surface of the article, the monolayer comprising a mixture of a first molecular species having a molecular structure promoting self-assembly at the surface with other first species in a tightly-packed manner preventing fluid to which the surface is exposed from communicating electrically with the surface, and a second molecular species that comprises a molecular wire.
183. A method as in A1, wherein the non-colloidal structure is a solid support.
184. A method as in 183, wherein the surface is a surface of an electrode.
185. A method as in 183, further comprising a ligand and an electroactive entity each forming part of a self-assembled monolayer at a surface of the colloid particle.
186. A method as in 185, the self-assembled monolayer including a species that enhances permeability of the self-assembled monolayer to electrons.
187. A method as in 186, wherein the species that enhances permeability to electrons comprises a conductive self-assembled monolayer-forming species.
188. A method as in 186, wherein the species that enhances permeability to electrons comprises a species that causes defect sites in the self-assembled monolayer
189. A method as in A104, wherein the electroactive entity comprises ferrocene dicarboxylic acid.
190. A method as in 185, wherein the ligand is linked to a self-assembled monolayer-forming species via a metal binding tag/metal/chelate linkage.
191. A method as in 183, wherein the solid support comprises a substantially planar substrate.
192. A method as in 183, wherein the surface of the article carries a self-assembled monolayer.
193. A method as in 192, wherein the self-assembled monolayer is a conductive self assembled monolayer.
194. A method as in 192, wherein the self-assembled monolayer includes a binding partner for an affinity tag.
195. A method as in 194, wherein the binding partner is a chelate able to coordinate a metal.
196. A method as in 194, wherein the binding partner for the affinity tag comprises glutathione or biotin.
197. A method as in 193, further comprising a chemical or biological agent fastened to the self-assembled monolayer via EDC/NHS coupling chemistry.
198. A method as in 193, comprising a plurality of chemical or biological binding partners fastened to the self-assembled monolayer and distributed randomly across the self assembled monolayer.
199. A method as in 193, wherein the self-assembled monolayer includes isolated regions each further comprising a unique chemical or biological binding partner fastened thereto.
200. A method as in 182, wherein the colloidal structure includes a chemical or biological agent fastened thereto that is a binding partner or is suspected of being a binding partner of a chemical or biological agent fastened to the surface of the article.
201. A method as in 200, wherein the colloidal particle includes and auxiliary signaling entity.
202. A method as in 200, further comprising a self-assembled monolayer on the surface of the colloidal particle.
203. A method as in 201, wherein the auxiliary signaling entity is an electroactive signaling entity.
204. A method as in 201, wherein the auxiliary signaling entity is a visible signaling entity.

## Claims

1. An article having a surface capable of facilitating the attachment of a biological or chemical agent, wherein the article is a nanoparticle, the surface of which is covered with a self-assembled monolayer.

2. An article as in claim 1 wherein the article comprises an auxiliary signaling entity.

3. An article as in claim 1 or 2 wherein the self-assembled monolayer facilitates the attachment of the biological or chemical agent via an affinity tag/binding partner linkage, wherein the affinity tag is on the biological or chemical agent and the binding partner is immobilized relative to the self-assembled monolayer.

4. An article as in claim 2 wherein the binding partner of the affinity tag is incorporated into the self-assembled monolayer.

5. An article as in claim 3 wherein the binding partner of the affinity tag is a metal chelate and the affinity tag is a poly-amino acid tag.

6. An article as in claim 4 wherein the binding partner of the affinity tag is nitrilo tri-acetic acid and the affinity tag is a poly-histidine tag.

7. An article as in claim 4 wherein glutathione is the binding partner of the affinity tag Glutathione-S-transferase.

8. An article as in claim 4 wherein biotin is the binding partner of the affinity tag streptavidin.

9. An article as in claim 1 or 2 wherein the self-assembled monolayer facilitates the attachment of the biological or chemical agent via covalent coupling.

10. An article as in claim 9 wherein the self-assembled monolayer bears reactive headgroups that facilitate covalent coupling of the biological or chemical agent.

11. An article as in claim 10 wherein the reactive headgroups are carboxylates and the biological or chemical agent is coupled via EDC/NHS chemistry.

12. A method comprising:
allowing the article of claim 1 or 2 to become immobilized with respect to a non-colloidal structure; and
determining the immobilization of the article relative to the non-colloidal structure.

13. A method as in claim 12, further comprising a biological or chemical agent linked to or adapted for linkage to the non-colloidal structure and a putative binding partner of the biological or chemical agent linked to or adapted for linkage to the article, the allowing step comprising allowing the article to become immobilized relative to the non-colloidal structure via the agent and the binding partner, and determining the immobilization.

14. A method as in claim 10 or 11, wherein the non-colloidal structure is a bead, a magnetic bead, a planar substrate, a chip, a cell or bodily tissue.

15. A kit comprising any of the articles of claims 1-11 and instructions for linking a biological or chemical agent to the articles.
